# EUROPÄISCHE PATENTSCHRIFT

(11) **EP 3 394 036 B1**
(45) Veröffentlichungstag und Bekanntmachung des Hinweises auf die Patenterteilung: **29.07.2020**
(21) Anmeldenummer: 16804998.9
(22) Anmeldetag: 28.11.2016
(51) Int. Cl.: C07D 209/94, C07D 221/18, C07D 223/22, C07D 279/36, H01L 51/50, C07D 209/80, C07D 221/08, C07D 223/24, C07D 265/38, C07D 279/22, H01L 51/00

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(30) Priorität: 21.12.2015 EP 15201728
(43) Veröffentlichungstag der Anmeldung: 31.10.2018
(73) Patentinhaber: Merck Patent GmbH, 64293 Darmstadt (DE)
(72) Erfinder: RIEDMUELLER, Stefan, 60488 Frankfurt am Main (DE); KAUFHOLD, Oliver, 64297 Darmstadt (DE); MEYER, Sebastian, 63741 Aschaffenburg (DE)
(86) Internationale Anmeldenummer: PCT/EP2016/002011
(87) Internationale Veröffentlichungsnummer: WO 2017/108152

(56) Entgegenhaltungen:
- WO-A1-2012/048781
- WO-A1-2013/083216
- DE-A1-102006 031 990

## Beschreibung

Die vorliegende Erfindung betrifft Monobenzoindenofluoren-Verbindungen, die Verwendung der Verbindungen in elektronischen Vorrichtungen, elektronische Vorrichtungen enthaltend die Verbindungen, sowie Verfahren zur Herstellung der Verbindungen.

Es ist aktuell von Interesse, Verbindungen zu entwickeln, mit denen verbesserte Eigenschaften von elektronischen Vorrichtungen in einem oder mehreren relevanten Punkten erzielt werden können, wie beispielsweise Leistungseffizienz, Lebensdauer und Farbkoordinaten des emittierten Lichts.

Unter dem Begriff elektronische Vorrichtung werden gemäß der vorliegenden Anmeldung allgemein elektronische Vorrichtungen verstanden, welche organische Materialien enthalten. Insbesondere werden darunter verstanden organische integrierte Schaltungen (OICs), organische Feld-Effekt-Transistoren (OFETs), organische Dünnfilmtransistoren (OTFTs), organische lichtemittierende Transistoren (OLETs), organische Solarzellen (OSCs), organische optische Detektoren, organische Photorezeptoren, organische Feld-Quench-Devices (OFQDs), organische lichtemittierende elektrochemische Zellen (OLECs), organische Laserdioden (O-Laser) und organische Elektrolumineszenzvorrichtungen (OLEDs).

Von besonderem Interesse ist die Bereitstellung von Verbindungen zur Verwendung in den zuletzt genannten, als OLEDs bezeichneten elektronischen Vorrichtungen. Der allgemeine Aufbau sowie das Funktionsprinzip von OLEDs ist dem Fachmann bekannt und unter anderem in US 4539507, US 5151629, EP 0676461 und WO 1998/27136 beschrieben.

Betreffend die Leistungsdaten von OLEDs sind noch weitere Verbesserungen erforderlich, insbesondere in Hinblick auf eine breite kommerzielle Verwendung, beispielsweise in Displays oder als Lichtquellen. Von besonderer Bedeutung sind in diesem Zusammenhang die Lebensdauer, die Effizienz und die Betriebsspannung der OLEDs sowie die realisierten Farbwerte. Insbesondere bei blau emittierenden OLEDs besteht Verbesserungspotential bezüglich der Lebensdauer der Vorrichtungen.

Von großer Bedeutung ist in diesem Zusammenhang die Wahl der Verbindung, welche als emittierende Verbindung in der OLED eingesetzt wird.

Im Stand der Technik sind hierzu eine Vielzahl an Verbindungen bekannt, insbesondere Arylamine mit einer oder mehreren kondensierten Arylgruppen.

Beispielhaft sind hier die in WO 2008/006449 offenbarten Verbindungen zu nennen, welche auf einem Indenofluoren-Gerüst beruhen, bei dem eine der Phenylgruppen zu einer größeren Arylgruppe erweitert ist, beispielsweise zu einer Naphthylgruppe. Außerdem offenbart WO 2013/083216 Spiro-Dihydroacridin-Derivate, die eine Benzindenofluoren-Gruppe umfassen. WO 2012/048781 offenbart ein Triphenylenderivat, das eine Benzindenofluoren-Gruppe umfasst.

Die in den oben genannten Anmeldungen offenbarten Verbindungen sind zwar wertvolle funktionelle Verbindungen, sie sind jedoch noch bezüglich bestimmer Aspekte verbesserbar. Insbesondere gibt es aufgrund der immer weiter steigenden Anforderungen kontinuierlichen Verbesserungsbedarf in Bezug auf Leistungseffizienz und Lebensdauer.

Überraschend wurde gefunden, dass die im Folgenden definierten neuen Verbindungen, welche eine N-heterocyclische Gruppe aufweisen, die an ein Monobenzindenofluoren-Grundgerüst gebunden ist, Verbesserungen der Leistungseffizienz und der Lebensdauer der OLEDs bewirken.

Gegenstand der vorliegenden Anmeldung sind damit Verbindungen gemäß Formel (I) 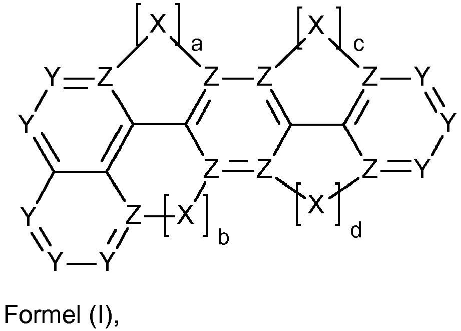 wobei für die auftretenden Variablen gilt:
- Y: ist bei jedem Auftreten gleich oder verschieden gewählt aus CR¹ und N;
- Z: ist gleich C, falls an die Gruppe Z eine Brücke X gebunden ist, und ist gleich Y, falls an die Gruppe Z keine Brücke X gebunden ist;
- X: ist bei jedem Auftreten gleich oder verschieden eine bivalente Brücke, ausgewählt aus C(R¹)₂ und Si(R¹)₂,
- R¹: ist bei jedem Auftreten gleich oder verschieden gewählt aus H, D, F, C(=O)R², CN, Si(R²)₃, N(R²)₂, P(=O)(R²)₂, OR², S(=O)R², S(=O)₂R², geradkettigen Alkyl- oder Alkoxygruppen mit 1 bis 20 C-Atomen, verzweigten oder cyclischen Alkyl- oder Alkoxy-gruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R² substituiert sein können; und wobei eine oder mehrere CH₂-Gruppen in den genannten Alkyl-, Alkoxy-, Alkenyl- und Alkinylgruppen durch -R²C=CR²-, -C=C-, Si(R²)₂, C=O, C=NR², -C(=O)O-, -C(=O)NR²- , NR², P(=O)(R²), -O-, -S-, SO oder SO₂ ersetzt sein können;
- R²: ist bei jedem Auftreten gleich oder verschieden gewählt aus H, D, F, CN, Alkylgruppen mit 1 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R² miteinander verknüpft sein können und einen Ring bilden können; und wobei die genannten Alkylgruppen, aromatischen Ringsysteme und heteroaromatischen Ringsysteme mit F oder CN substituiert sein können;
- a, b, c,: d ist bei jedem Auftreten gleich oder verschieden 0 oder 1, mit der Maßgabe, dass a + b = 1 ist und c + d = 1 ist, wobei a = 0 bzw. b = 0 bzw. c = 0 bzw. d = 0 jeweils bedeutet, dass die entsprechende Brücke X nicht vorhanden ist;
wobei mindestens ein Y vorliegt, bei dem anstelle von R¹ eine Einheit der Formel (N) gebunden ist, 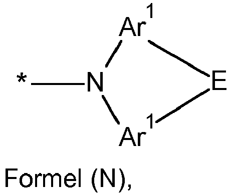 bei der die mit Stern gekennzeichnete Bindung die Bindung an Y markiert, und wobei die Formel (N) einer der folgenden Formeln (N-2) bis (N-12) entspricht: 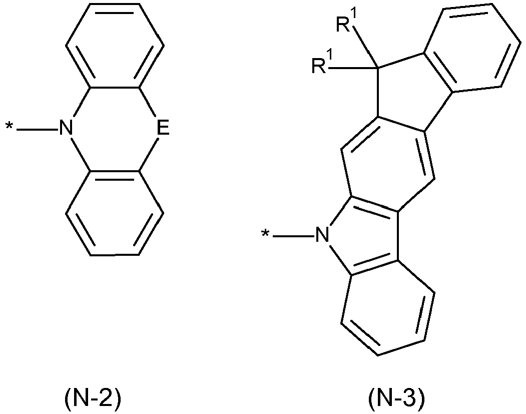 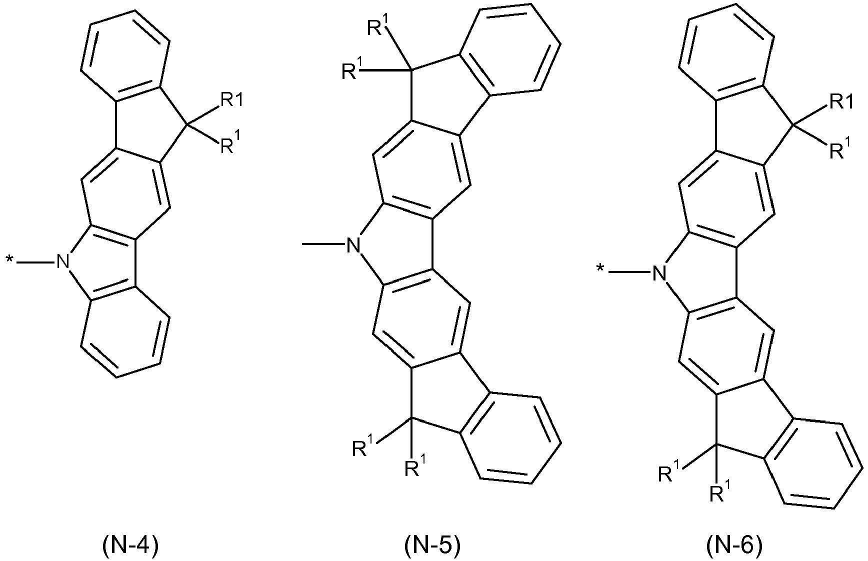 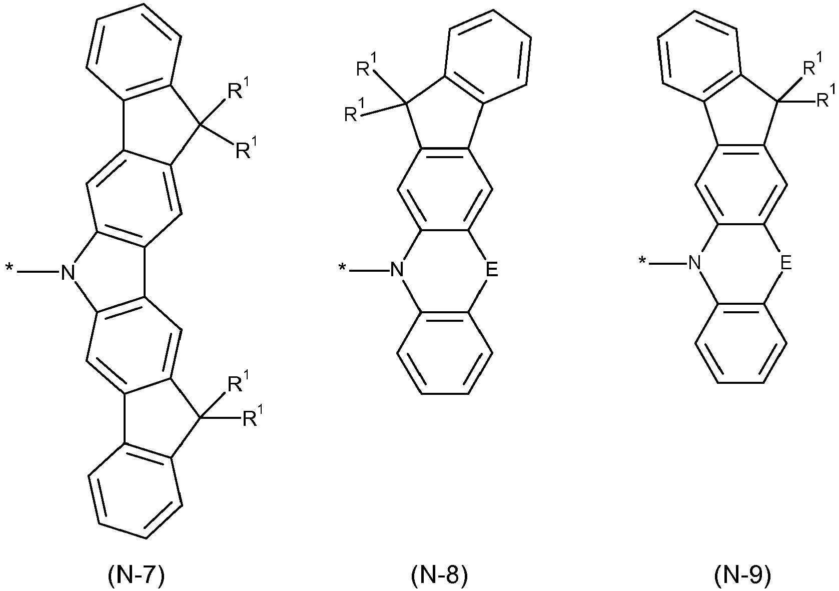 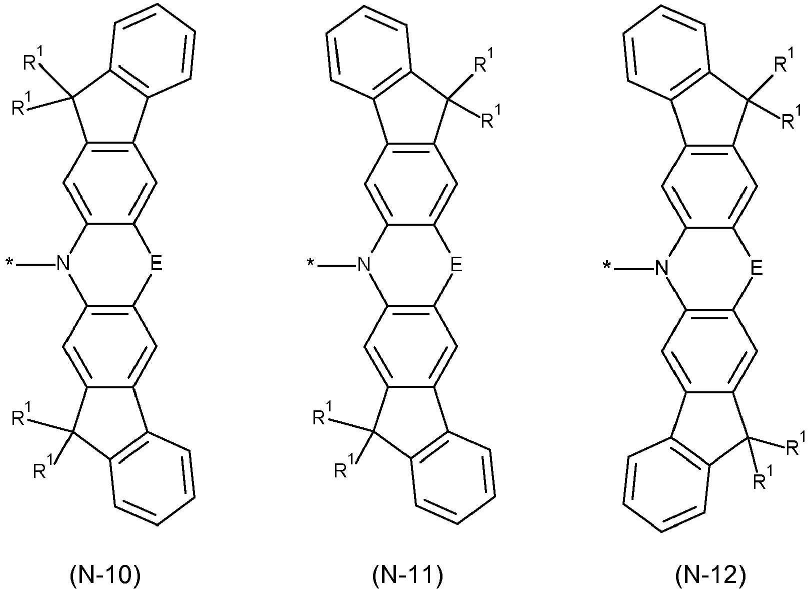 wobei die Einheiten der Formel (N) an allen unsubstituiert gezeichneten Positionen mit Resten R¹ substituiert sein können;
- Ar¹: ist bei jedem Auftreten gleich oder verschieden gewählt aus aromatischen Ringsystemen wie in (N-2) to (N-12) dargestellt, welche mit einem oder mehreren Resten R¹ substituiert sein können;
- E: ist bei jedem Auftreten gleich oder verschieden gewählt aus den folgenden divalenten Gruppen

-C(R¹)₂-C(R¹)₂- (E-2)

-C(R¹)₂-C(R¹)₂-C(R¹)₂- (E-3)

-CR¹=CR¹- (E-4)
wobei R¹ definiert ist wie oben.

Eine Arylgruppe im Sinne dieser Erfindung enthält 6 bis 40 aromatische Ringatome, von denen keines ein Heteroatom darstellt. Unter einer Arylgruppe im Sinne dieser Erfindung wird entweder ein einfacher aromatischer Cyclus, also Benzol, oder ein kondensierter aromatischer Polycyclus, beispielsweise Naphthalin, Phenanthren oder Anthracen, verstanden. Ein kondensierter aromatischer Polycyclus besteht im Sinne der vorliegenden Anmeldung aus zwei oder mehr miteinander kondensierten einfachen aromatischen Cyclen. Unter Kondensation zwischen Cyclen ist dabei zu verstehen, dass die Cyclen mindestens eine Kante miteinander teilen.

Eine Heteroarylgruppe im Sinne dieser Erfindung enthält 5 bis 40 aromatische Ringatome, von denen mindestens eines ein Heteroatom darstellt. Die Heteroatome der Heteroarylgruppe sind bevorzugt ausgewählt aus N, O und S. Unter einer Heteroarylgruppe im Sinne dieser Erfindung wird entweder ein einfacher heteroaromatischer Cyclus, beispielsweise Pyridin, Pyrimidin oder Thiophen, oder ein kondensierter heteroaromatischer Polycyclus, beispielsweise Chinolin oder Carbazol, verstanden. Ein kondensierter heteroaromatischer Polycyclus besteht im Sinne der vorliegenden Anmeldung aus zwei oder mehr miteinander kondensierten einfachen heteroaromatischen Cyclen. Unter Kondensation zwischen Cyclen ist dabei zu verstehen, dass die Cyclen mindestens eine Kante miteinander teilen.

Unter einer Arylen- bzw. Heteroarylengruppe wird entsprechend die von einer Aryl- bzw. Heteroarylgruppe abgeleitete divalente Einheit verstanden.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Triphenylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung enthält 6 bis 40 C-Atome im Ringsystem und umfasst keine Heteroatome als aromatische Ringatome. Ein aromatisches Ringsystem im Sinne dieser Erfindung enthält daher keine Heteroarylgruppen. Unter einem aromatischen Ringsystem im Sinne dieser Erfindung soll ein System verstanden werden, das nicht notwendigerweise nur Arylgruppen enthält, sondern in dem auch mehrere Arylgruppen durch eine Einfachbindung oder durch eine nicht-aromatische Einheit, wie beispielsweise ein oder mehrere wahlweise substituierte C-, Si-, N-, O- oder S-Atome, verbunden sein können. Dabei umfasst die nicht-aromatische Einheit bevorzugt weniger als 10 % der von H verschiedenen Atome, bezogen auf die Gesamtzahl der von H verschiedenen Atome des Systems. So sollen beispielsweise auch Systeme wie 9,9'-Spirobifluoren, 9,9'-Diarylfluoren, Triarylamin, Diarylether und Stilben als aromatische Ringsysteme im Sinne dieser Erfindung verstanden werden, und ebenso Systeme, in denen zwei oder mehr Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Arylgruppen über Einfachbindungen miteinander verknüpft sind, als aromatische Ringsysteme im Sinne dieser Erfindung verstanden, wie beispielsweise Systeme wie Biphenyl und Terphenyl.

Ein heteroaromatisches Ringsystem im Sinne dieser Erfindung enthält 5 bis 40 aromatische Ringatome, von denen mindestens eines ein Heteroatom darstellt. Die Heteroatome des heteroaromatischen Ringsystems sind bevorzugt ausgewählt aus N, O und/oder S. Ein heteroaromatisches Ringsystem entspricht der oben genannten Definition eines aromatischen Ringsystems, weist jedoch mindestens ein Heteroatom als eines der aromatischen Ringatome auf. Es unterscheidet sich dadurch von einem aromatischen Ringsystem im Sinne der Definition der vorliegenden Anmeldung, welches gemäß dieser Definition kein Heteroatom als aromatisches Ringatom enthalten kann.

Unter einem aromatischen Ringsystem mit 6 bis 40 aromatischen Ringatomen oder einem heteroaromatischen Ringsystem mit 5 bis 40 aromatischen Ringatomen, werden insbesondere Gruppen verstanden, die abgeleitet sind von den oben unter Arylgruppen und Heteroarylgruppen genannten Gruppen sowie von Biphenyl, Terphenyl, Quaterphenyl, Fluoren, Spirobifluoren, Dihydrophenanthren, Dihydropyren, Tetrahydropyren, Indenofluoren, Truxen, Isotruxen, Spirotruxen, Spiroisotruxen, Indenocarbazol, oder von Kombinationen dieser Gruppen.

Im Rahmen der vorliegenden Erfindung werden unter einer geradkettigen Alkylgruppe mit 1 bis 20 C-Atomen bzw. einer verzweigten oder cyclischen Alkylgruppe mit 3 bis 20 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden.

Unter einer Alkoxy- oder Thioalkylgruppe mit 1 bis 20 C-Atomen, in der auch einzelne H-Atome oder CH₂-Gruppen durch die oben bei der Definition der Reste genannten Gruppen substituiert sein können, werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Bevorzugt liegt in der Verbindung der Formel (I) genau ein Y vor, bei dem anstelle von R¹ eine Einheit der Formel (N) gebunden ist.

Gemäß einer bevorzugten Ausführungsform ist Ar¹ bei jedem Auftreten gleich gewählt.

Die optional mit R¹ substituierten Arylengruppen sind bevorzugt optional mit R¹ substituierte Phenylengruppen, besonders bevorzugt optional mit R¹ substituierte 1,3-Phenylengruppen.

Bevorzugt sind höchstens drei Gruppen Y je aromatischem Sechsring gleich N, besonders bevorzugt höchstens 2. Ganz besonders bevorzugt sind alle Gruppen Y gleich CR¹.

Bevorzugt ist X gleich C(R¹)₂.

Bevorzugt ist R¹ bei jedem Auftreten gleich oder verschieden gewählt aus H, D, F, CN, Si(R²)₃, N(R²)₂, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit einem oder mehreren Resten R² substituiert sein können; und wobei in den genannten Alkyl- oder Alkoxygruppen eine oder mehrere CH₂-Gruppen durch -C=C-, -R²C=CR²-, Si(R²)₂, C=O, C=NR², -NR²-, -O-, -S-, -C(=O)O- oder -C(=O)NR²- ersetzt sein können.

Weiterhin bevorzugt ist es, dass Reste R¹ in Einheiten X bei jedem Auftreten gleich oder verschieden gewählt sind aus H, D, F, CN, geradkettigen Alkylgruppen mit 1 bis 12 C-Atomen, verzweigten oder cyclischen Alkylgruppen mit 3 bis 12 C-Atomen, aromatischen Ringsystemen mit 6 bis 20 aromatischen Ringatomen, und heteroaromatischen Ringsystemen mit 5 bis 20 aromatischen Ringatomen; wobei die genannten Alkylgruppen, die genannten aromatischen Ringsysteme und die genannten heteroaromatischen Ringsysteme jeweils mit einem oder mehreren Resten R² substituiert sein können.

Besonders bevorzugt sind Reste R¹ in Einheiten X bei jedem Auftreten gleich oder verschieden gewählt aus geradkettigen Alkylgruppen mit 1 bis 12 C-Atomen, verzweigten oder cyclischen Alkylgruppen mit 3 bis 12 C-Atomen, und aromatischen Ringsystemen mit 6 bis 20 aromatischen Ringatomen; wobei die genannten Alkylgruppen und die genannten aromatischen Ringsysteme jeweils mit einem oder mehreren Resten R² substituiert sein können.

Reste R¹ in Einheiten Y sind bevorzugt bei jedem Auftreten gleich oder verschieden gewählt aus H, D, F, CN, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R² substituiert sein können.

Besonders bevorzugt sind Reste R¹ in Einheiten Y gleich H.

Bevorzugte Ausführungsformen der Formel (I) entsprechen den folgenden Formeln (I-1) bis (I-4) 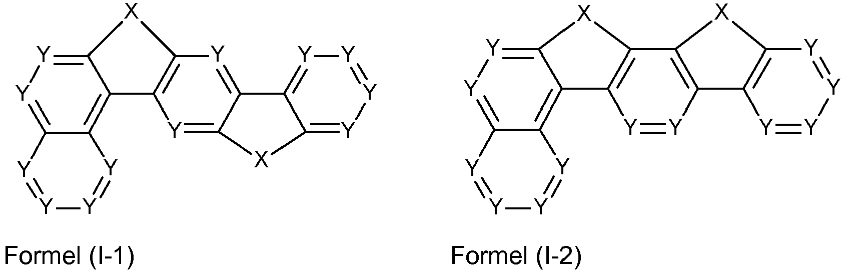 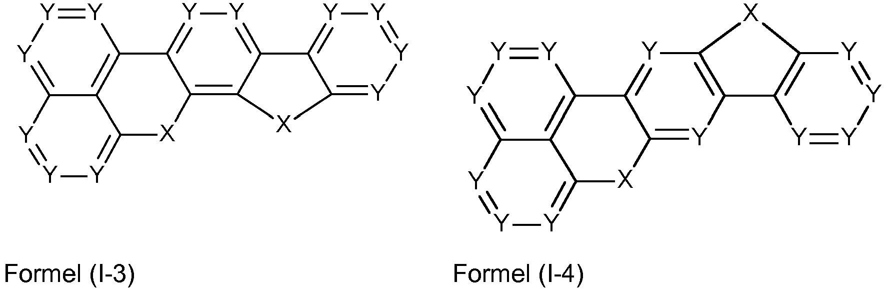 wobei die auftretenden Variablen definiert sind wie oben, und wobei mindestens ein Y vorliegt, bei dem anstelle von R¹ eine Einheit der Formel (N), wie oben definiert, gebunden ist.

Bevorzugt unter den Formeln (I-1) bis (I-4) ist die Formel (I-1).

Weiterhin bevorzugt ist die Kombination der bevorzugten Formeln für das Grundgerüst gemäß Formeln (I-1) bis (I-4), insbesondere der Formel (I-1), mit einer der bevorzugten Formeln für die Gruppe N, gemäß Formeln (N-1) bis (N-4).

Besonders bevorzugte Ausführungsformen der Formel (I) entsprechen den folgenden Formeln (I-1-1) bis (I-4-1) 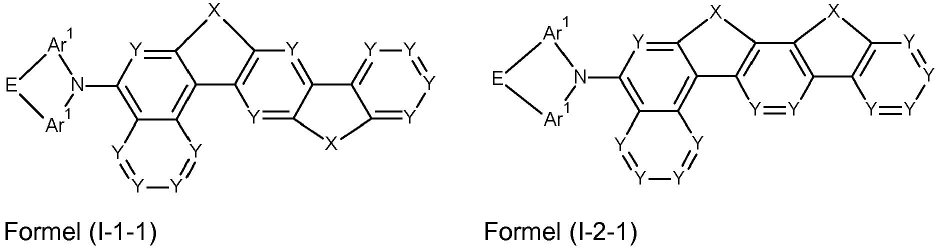 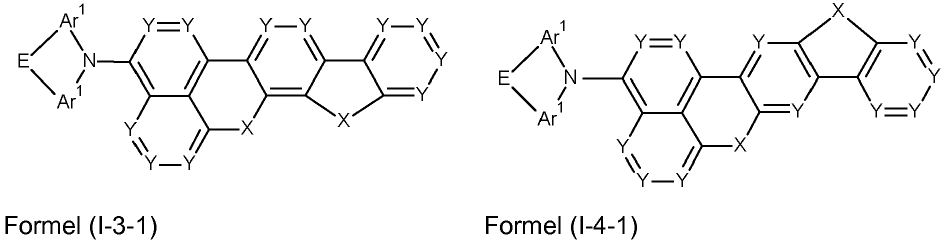 wobei die auftretenden Variablen definiert sind wie oben.

Bevorzugt unter den Formeln (I-1-1) bis (I-4-1) ist die Formel (I-1-1).

Weiterhin bevorzugt ist die Kombination der bevorzugten Formeln für das Grundgerüst gemäß Formeln (I-1-1) bis (I-4-1), insbesondere der Formel (I-1-1), mit einer der bevorzugten Formeln für die Gruppe N, gemäß Formeln (N-1) bis (N-4).

Beispiele für Verbindungen der Formel (I) sind in der folgenden Tabelle abgebildet:

| | |
|---|---|
| 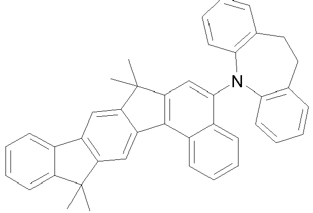 | 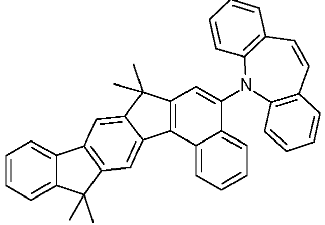 |
| (1) | (2) |
| 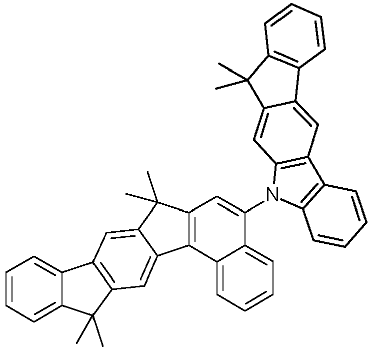 | 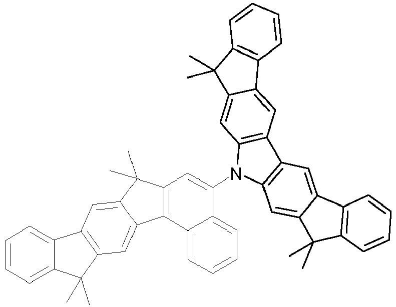 |
| (3) | (4) |
| 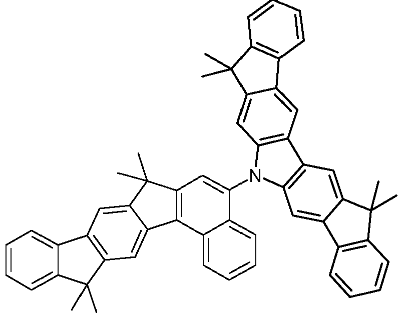 | 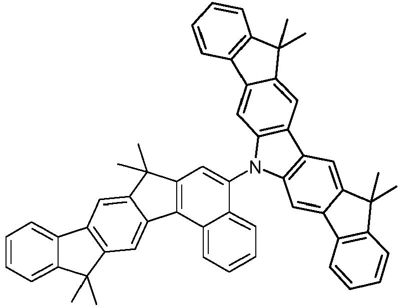 |
| (5) | (6) |
| 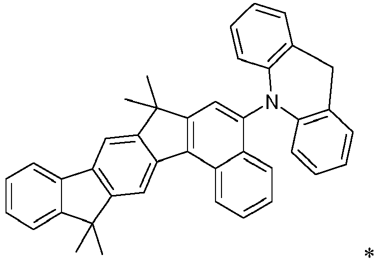 | 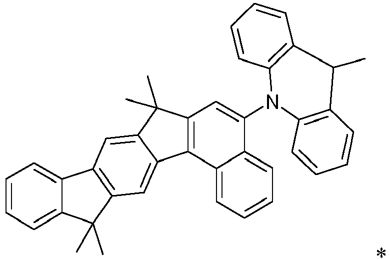 |
| 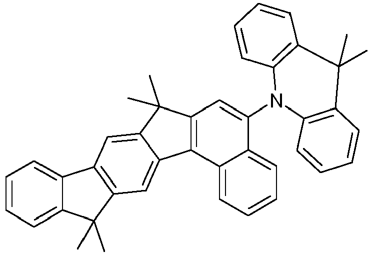 | 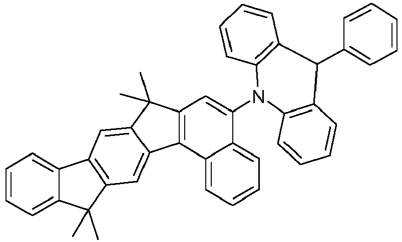 |
| (7)* | (8)* |
| 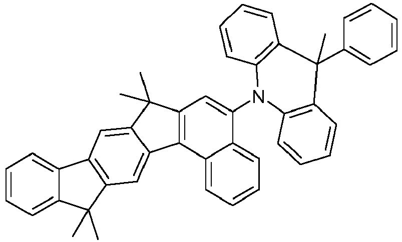 | 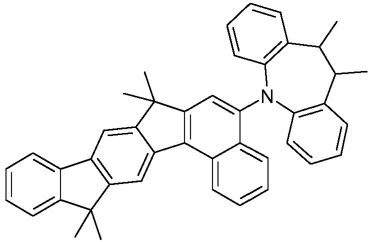 |
| (9)* | (10) |
| 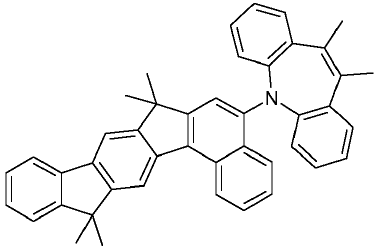 | 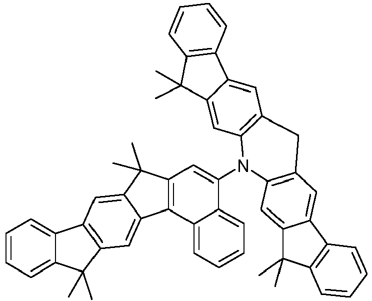 |
| (11) | (12)* |
| 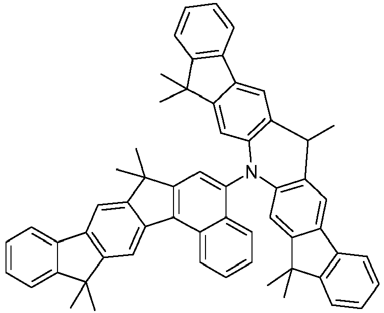 | 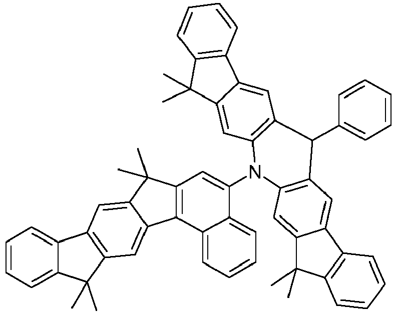 |
| (13)* | (14)* |
| 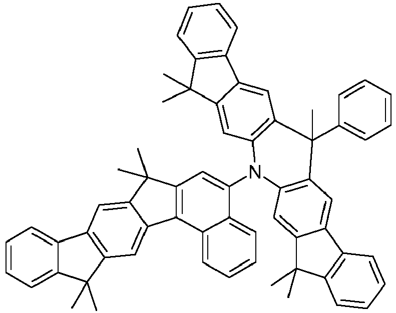 | 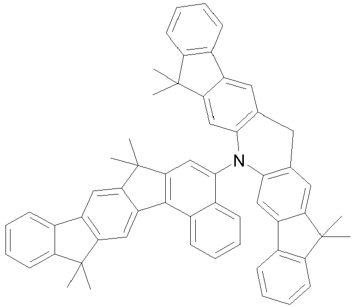 |
| (15)* | (16)* |
| 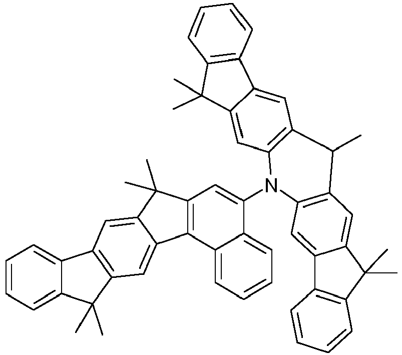 | 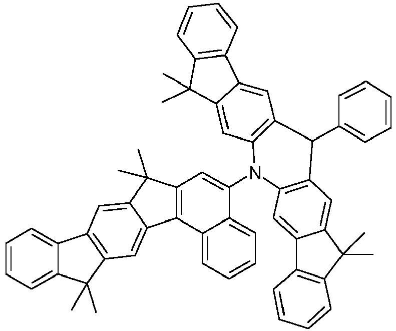 |
| (17)* | (18)* |
| 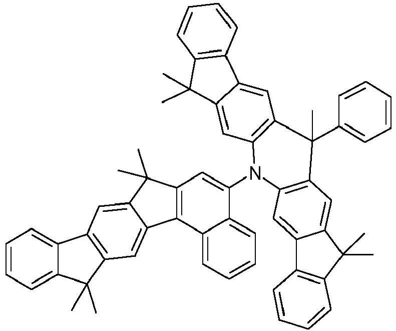 | 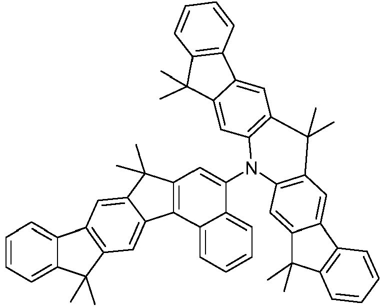 |
| (19)* | (20)* |
| 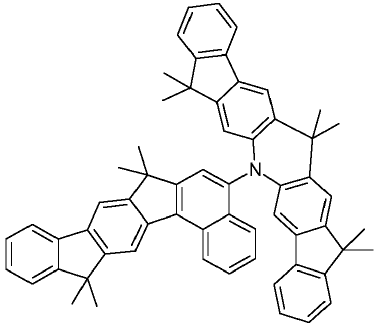 | 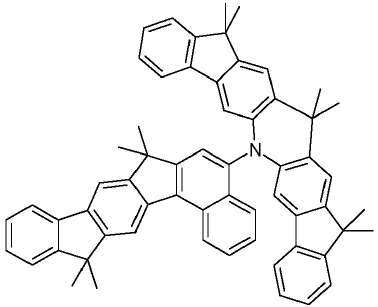 |
| (21)* | (22)* |
| 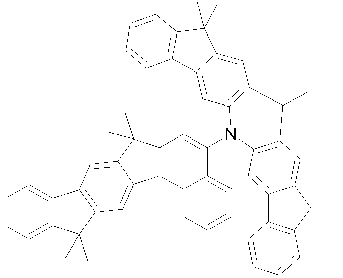 | 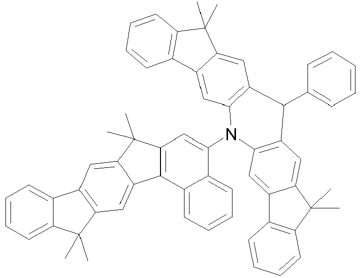 |
| (23)* | (24)* |
| 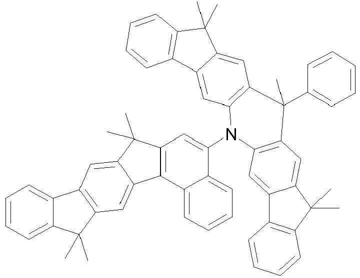 | 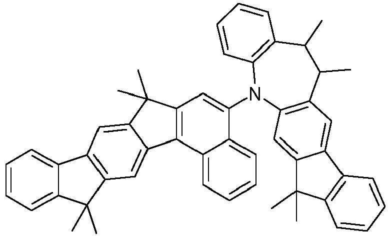 |
| (25)* | (26) |
| 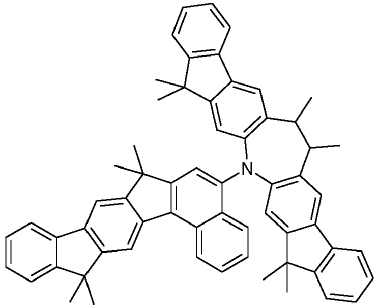 | 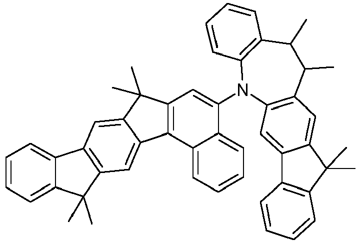 |
| (27) | (28) |
| 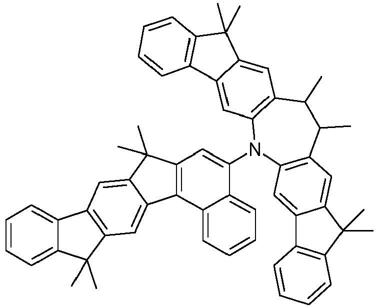 | 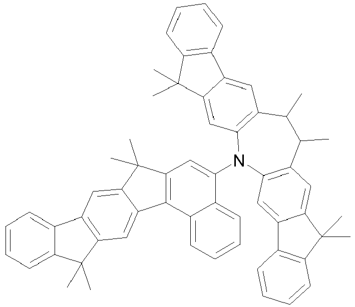 |
| (29) | (30) |
| 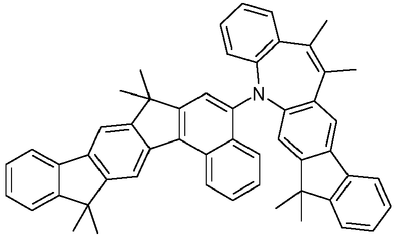 | 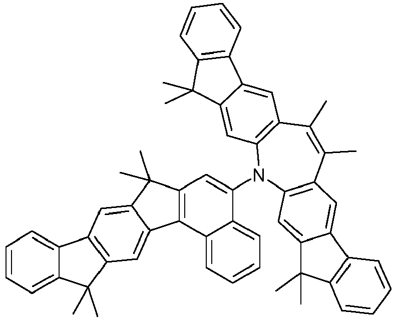 |
| (31) | (32) |
| 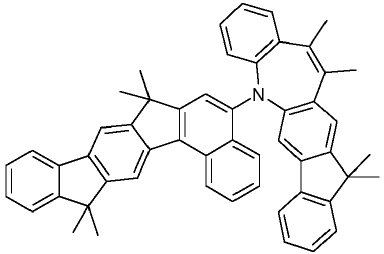 | 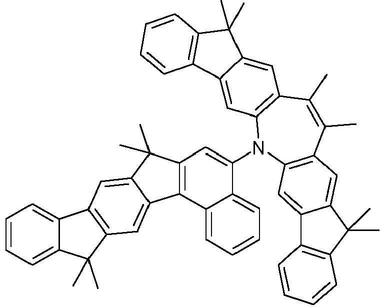 |
| (33) | (34) |
| 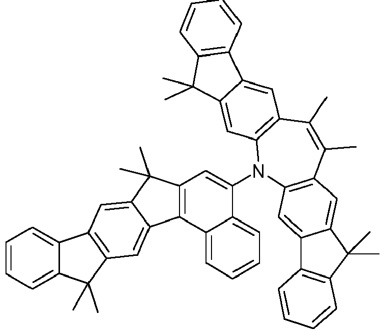 | 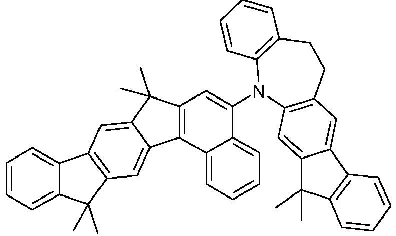 |
| (35) | (36) |
| 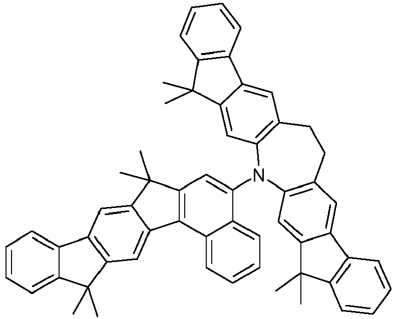 | 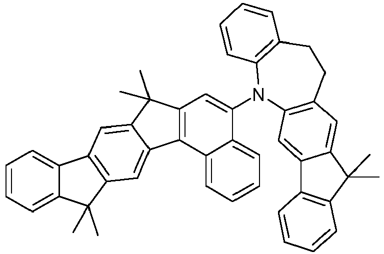 |
| (37) | (38) |
| 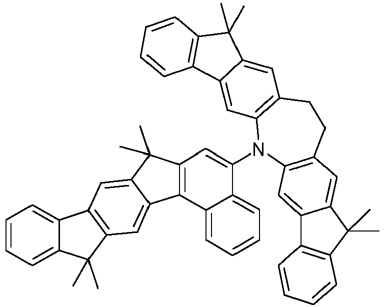 | 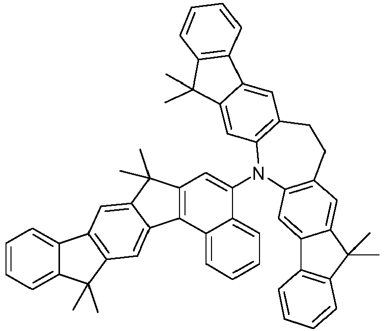 |
| (39) | (40) |
| 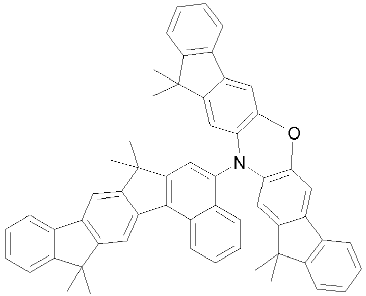 | 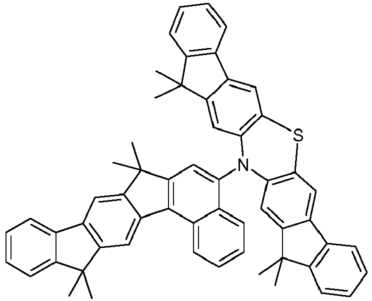 |
| (41)* | (42)* |
| 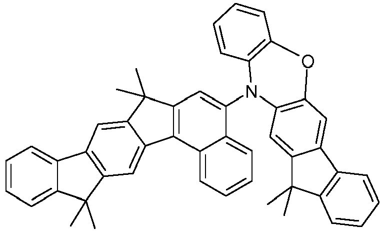 | 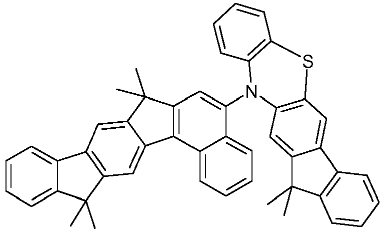 |
| (43)* | (44)* |
| 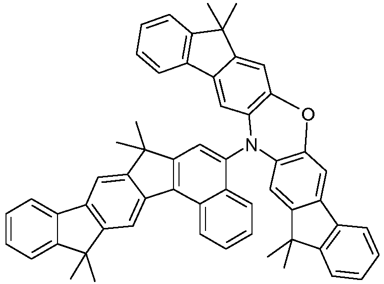 | 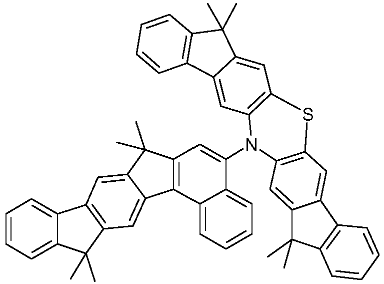 |
| (45)* | (46)* |
| 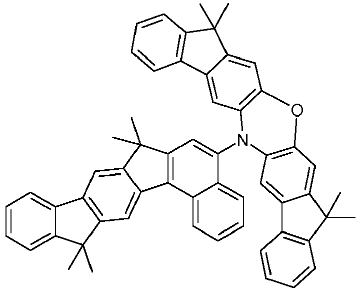 | 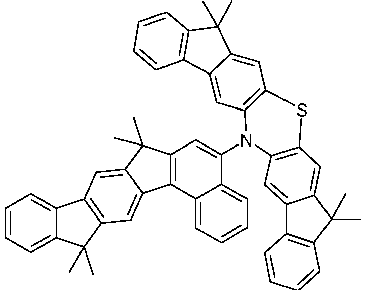 |
| (47)* | (48)* |

| | |
|---|---|
| ** nicht erfindungsgemäß* | |

Die Verbindungen der Formel (I) können mittels Standardreaktionen der organischen Synthesechemie hergestellt werden, insbesondere durch säurekatalysierte Ringschlussreaktionen von tertiären Alkoholen, durch Suzuki-Kupplungsreaktionen und durch Buchwald-Kupplungsreaktionen.

Ein geeignetes Syntheseverfahren zur Herstellung einer Verbindung der Formel (I) ist im Folgenden gezeigt (Schema 1). Weitere Detailangaben hierzu finden sich in den Ausführungsbeispielen. 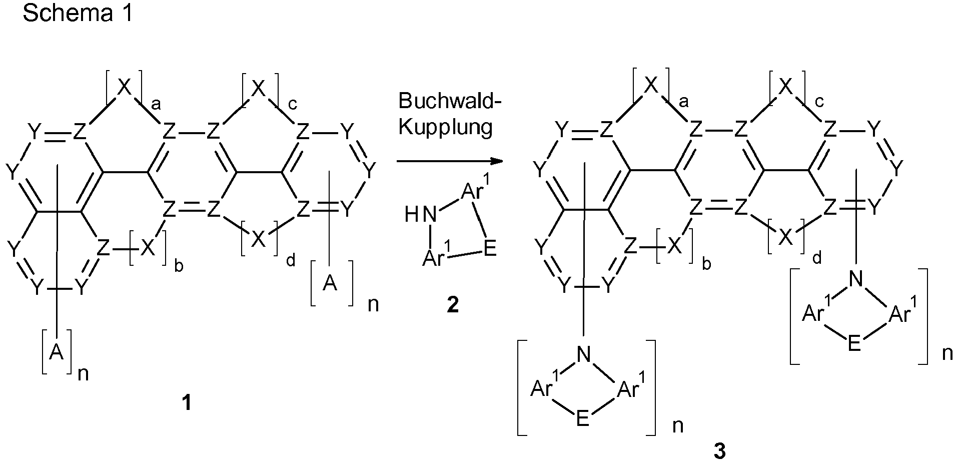 wobei die auftretenden Variablen definiert sind wie oben, und weiterhin gilt:
A = reaktive Gruppe, bevorzugt Halogen, Sn-Organyl, Si-Organyl, Mesylat oder Triflat, besonders bevorzugt Cl, Br oder I.
n= 0, 1 oder 2, bevorzugt 0 oder 1, wobei die Summe aller n bevorzugt gleich 1 ist.

Gemäß dem gezeigten Verfahren wird ein Monobenzindenofluoren-Derivat 1, welches eine oder mehrere reaktive Gruppen enthält, in einer Buchwald-Reaktion mit einem sekundären Amin 2 umgesetzt. Dabei wird die Zielverbindung 3 gemäß Formel (I) erhalten. Das genannte Edukt 1 kann wie in WO 2008/006449 A1 beschrieben hergestellt werden. Die Edukte 2 sind entweder literaturbekannt wie das Dimethylindenocarbazol, dessen Synthese in WO 2011/050888 A1 auf S. 73-75 offenbart ist, oder können gemäß dem Fachmann bekannten Verfahren hergestellt werden.

Weiterer Gegenstand der vorliegenden Erfindung ist damit ein Verfahren zur Herstellung einer Verbindung der Formel (I), dadurch gekennzeichnet, dass ein Monobenzindenofluoren-Derivat, welches eine oder mehrere reaktive Gruppen umfasst, in einer übergangsmetallkatalysierten Kupplungsreaktion mit einem Amin umgesetzt wird.

Bevorzugt ist die übergangsmetallkatalysierte Kupplungsreaktion eine Buchwald-Kupplung.

Die reaktiven Gruppen sind bevorzugt gewählt aus Cl, Br, I, Sn-Organylen, Si-Organylen, Mesylaten und Triflaten. Bevorzugt umfasst das Monobenzindenofluoren-Derivat genau eine reaktive Gruppe.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere enthaltend eine oder mehrere Verbindungen gemäß Formel (I), wobei die Bindung(en) zum Polymer, Oligomer oder Dendrimer an beliebigen, in Formel (I) mit R¹ oder R² substituierten Positionen lokalisiert sein können. Je nach Verknüpfung der Verbindung gemäß Formel (I) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß Formel (I) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I) in Oligomeren, Dendrimeren und Polymeren gelten dieselben Bevorzugungen wie oben für Verbindungen gemäß Formel (I) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 00/22026), Spirobifluorenen (z. B. gemäß EP 707020, EP 894107 oder WO 06/061181), Paraphenylenen (z. B. gemäß WO 1992/18552), Carbazolen (z. B. gemäß WO 04/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethyl-ether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (I) oder mindestens ein Polymer, Oligomer oder Dendrimer enthaltend mindestens eine Einheit gemäß Formel (I) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die Verbindungen gemäß Formel (I) eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Die Verbindung der Formel (I) kann in jeder Funktion in der organischen Elektrolumineszenzvorrichtung eingesetzt werden, beispielsweise als lochtransportierendes Material, als Matrixmaterial, als emittierendes Material, oder als elektronentransportierendes Material.

Weiterer Gegenstand der Erfindung ist daher die Verwendung einer Verbindung gemäß Formel (I) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Weiterer Gegenstand der Erfindung ist eine elektronische Vorrichtung, enthaltend mindestens eine Verbindung der Formel (I). Bevorzugt ist die elektronische Vorrichtung gewählt aus den oben angegebenen Vorrichtungen. Besonders bevorzugt ist eine organische Elektrolumineszenzvorrichtung, enthaltend Anode, Kathode und mindestens eine emittierende Schicht, dadurch gekennzeichnet, dass mindestens eine organische Schicht mindestens eine Verbindung gemäß Formel (I) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen.

Die Abfolge der Schichten der organischen Elektrolumineszenzvorrichtung ist bevorzugt die folgende: Anode-Lochinjektionsschicht-Lochtransportschicht-emittierende Schicht-Elektronentransportschicht-Elektroneninjektionsschicht-Kathode. Dabei müssen nicht alle der genannten Schichten vorhanden sein, und es können zusätzlich weitere Schichten vorhanden sein, beispielsweise eine Elektronenblockierschicht anodenseitig an die emittierende Schicht angrenzend, oder eine Lochblockierschicht kathodenseitig an die emittierende Schicht angrenzend.

Die erfindungsgemäße organische Elektrolumineszenzvorrichtung enthält bevorzugt zwei oder mehr Schichten mit lochtransportierender Funktion zwischen Anode und emittierender Schicht.

In einer lochtransportierenden Schicht kann das Lochtransportmaterial als Reinmaterial, d.h. in einem Anteil von 100 % eingesetzt werden, oder es kann in Kombination mit einer oder mehreren weiteren Verbindungen eingesetzt werden. Gemäß einer bevorzugten Ausführungsform enthält mindestens eine Lochtransportschicht der OLED zusätzlich zum Lochtransportmaterial einen oder mehrere p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugte Ausführungsformen von p-Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600 und WO 2012/095143 offenbarten Verbindungen.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues, grünes, gelbes, orangefarbenes oder rotes Licht emittieren. Insbesondere bevorzugt sind Dreischichtsysteme, also Systeme mit drei emittierenden Schichten, wobei bevorzugt mindestens eine dieser Schichten mindestens eine Verbindung gemäß Formel (I) enthält und wobei die drei Schichten blaue, grüne, gelbe, orangefarbene oder rote Emission zeigen (für den prinzipiellen Aufbau siehe z. B. WO 2005/011013). Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert. Alternativ und/oder zusätzlich können in einer derartigen organischen Elektrolumineszenzvorrichtung die erfindungsgemäßen Verbindungen auch in der Lochtransportschicht oder in einer anderen Schicht vorhanden sein. Die verschiedenen emittierenden Schichten können direkt aneinander grenzen, oder sie können durch nicht emittierende Schichten voneinander getrennt sein. Gemäß einer bevorzugten Ausführungsform der Erfindung ist eine weiß emittierende OLED eine sogenannte Tandem-OLED, das heißt es liegen zwei oder mehr vollständige OLED-Schichtabfolgen in der OLED vor, wobei die OLED-Schichtabfolgen jeweils Lochtransportschicht, emittierende Schicht und Elektronentransportschicht umfassen, die jeweils durch eine Ladungserzeugungsschicht (charge generation layer) voneinander getrennt sind.

Es ist bevorzugt, wenn die Verbindung gemäß Formel (I) in einer emittierenden Schicht eingesetzt wird. Insbesondere eignet sich die Verbindung gemäß Formel (I) zur Verwendung als emittierende Verbindung, besonders als blau emittierende Verbindung oder als im Nah-UV emittierende Verbindung.

Wenn die erfindungsgemäße Verbindung als emittierende Verbindung in einer emittierenden Schicht eingesetzt wird, wird sie bevorzugt in Kombination mit einem oder mehreren Matrixmaterialien eingesetzt. Unter einem Matrixmaterial wird dabei ein Material verstanden, welches in der emittierenden Schicht vorliegt, bevorzugt als Hauptkomponente, und welches im Betrieb der Vorrichtung nicht Licht emittiert.

Der Anteil der emittierenden Verbindung in der Mischung der emittierenden Schicht beträgt zwischen 0.1 und 50.0 %, bevorzugt zwischen 0.5 und 20.0 %, besonders bevorzugt zwischen 1.0 und 10.0 %. Entsprechend beträgt der Anteil des Matrixmaterials bzw. der Matrixmaterialien zwischen 50.0 und 99.9 %, bevorzugt zwischen 80.0 und 99.5 %, besonders bevorzugt zwischen 90.0 und 99.0 %.

Dabei wird unter den Angaben der Anteile in % im Rahmen der vorliegenden Anmeldung Vol.-% verstanden, wenn die Verbindungen aus der Gasphase aufgebracht werden, und es wird darunter Gew.-% verstanden, wenn die Verbindungen aus Lösung aufgebracht werden.

Im Folgenden sind allgemein bevorzugte Materialklassen zur Verwendung als entsprechende Funktionsmaterialien in den erfindungsgemäßen organischen Elektrolumineszenzvorrichtungen aufgeführt.

Als phosphoreszierende emittierende Verbindungen eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende emittierende Verbindungen Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele der oben beschriebenen phosphoreszierenden emittierenden Verbindungen können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen. Auch kann der Fachmann ohne erfinderisches Zutun weitere phosphoreszierende Komplexe in Kombination mit den erfindungsgemäßen Verbindungen in OLEDs einsetzen.

Bevorzugte fluoreszierende Emitter sind neben den erfindungsgemäßen Verbindungen ausgewählt aus der Klasse der Arylamine. Unter einem Arylamin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte Emitter sind Indenofluorenamine bzw. -diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen. Ebenfalls bevorzugt sind die in WO 2012/048780 und WO 2013/185871 offenbarten Pyren-Arylamine. Ebenfalls bevorzugt sind die in WO 2014/037077 offenbarten Benzoindenofluoren-Amine, die in WO 2014/106522 offenbarten Benzofluoren-Amine und die in WO 2014/111269 offenbarten erweiterten Indenofluorene.

Bevorzugte Matrixmaterialien zur Verwendung in Kombination mit fluoreszierenden emittierenden Verbindungen sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Besonders bevorzugte Matrixmaterialien zur Verwendung in Kombination mit der Verbindung der Formel (I) in der emittierenden Schicht sind in der folgenden Tabelle abgebildet.

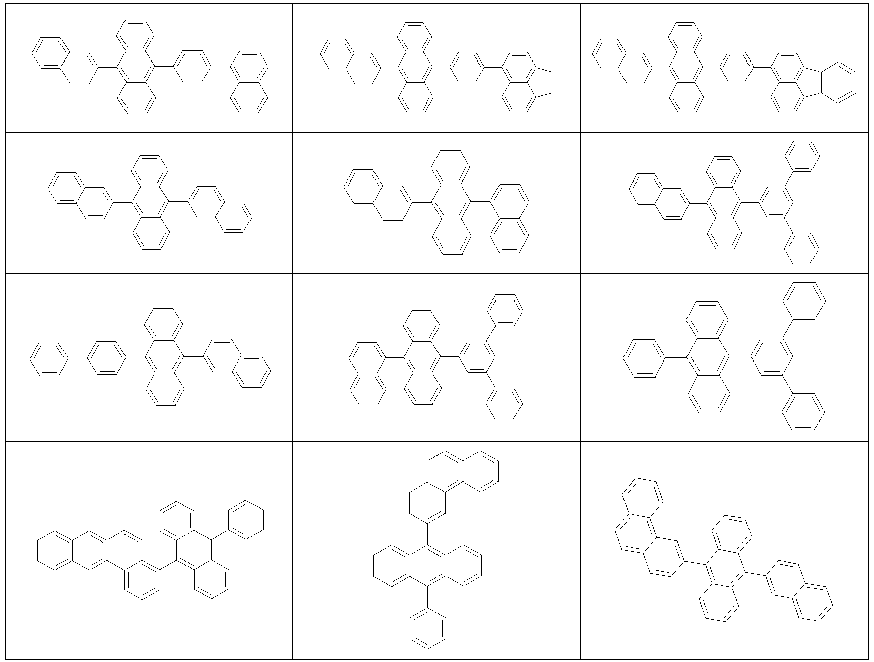

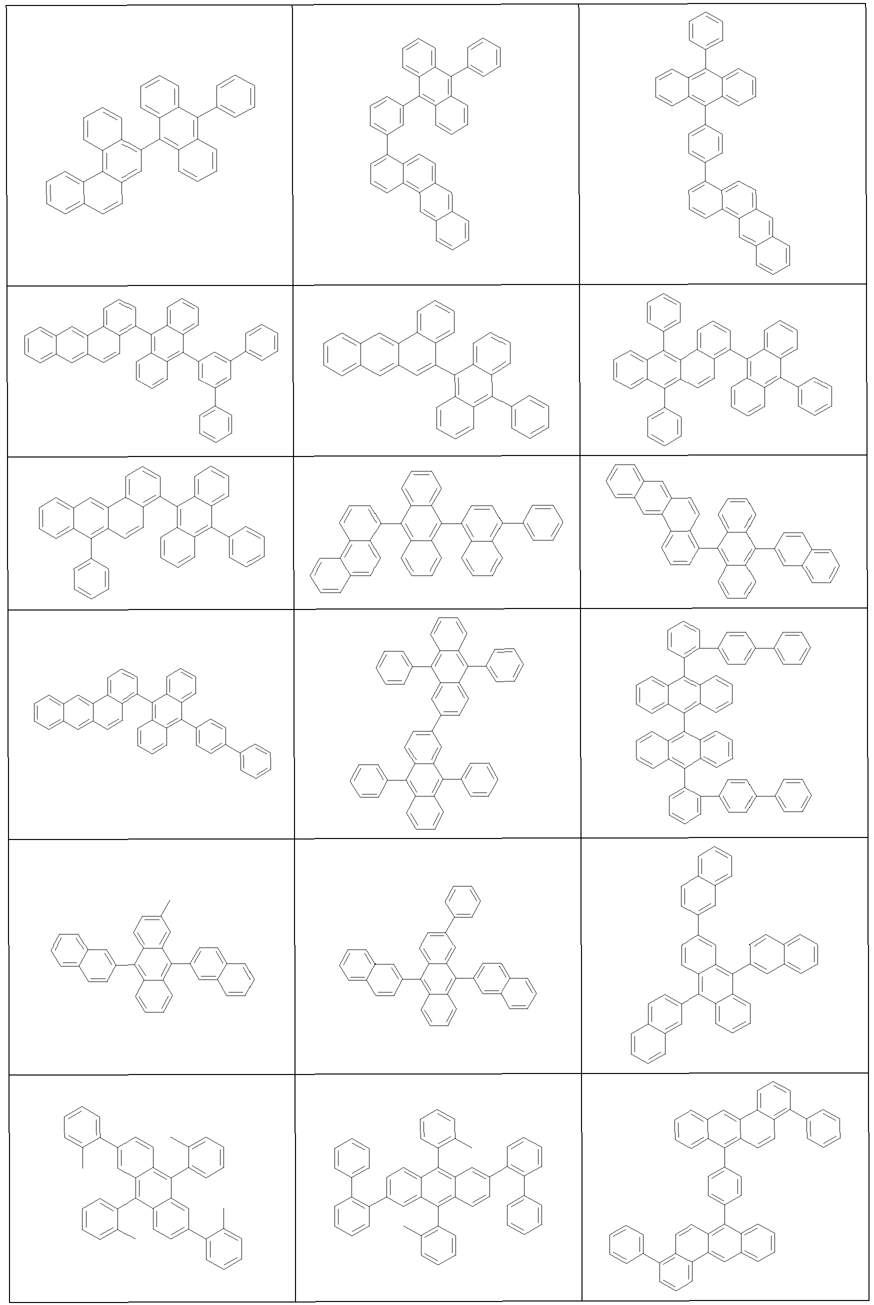

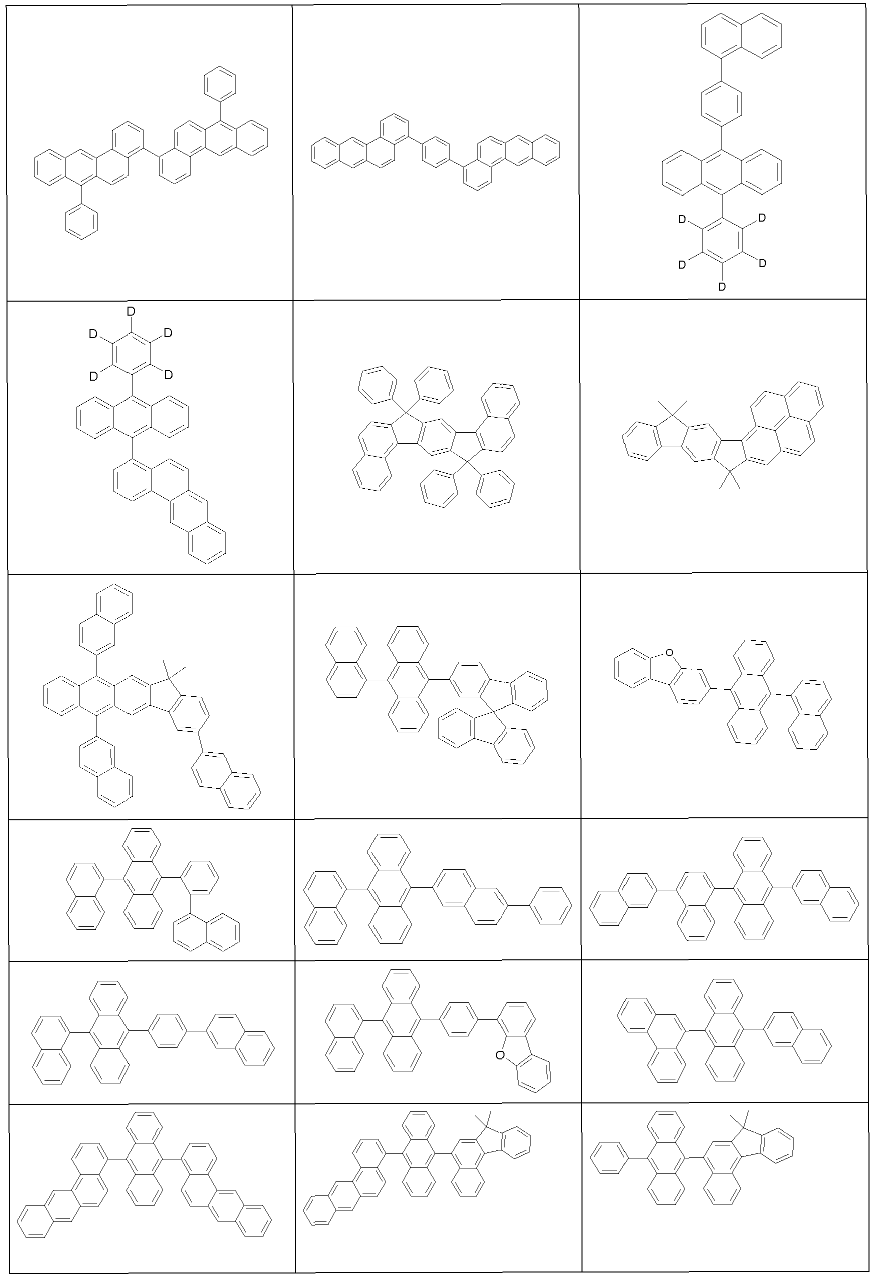

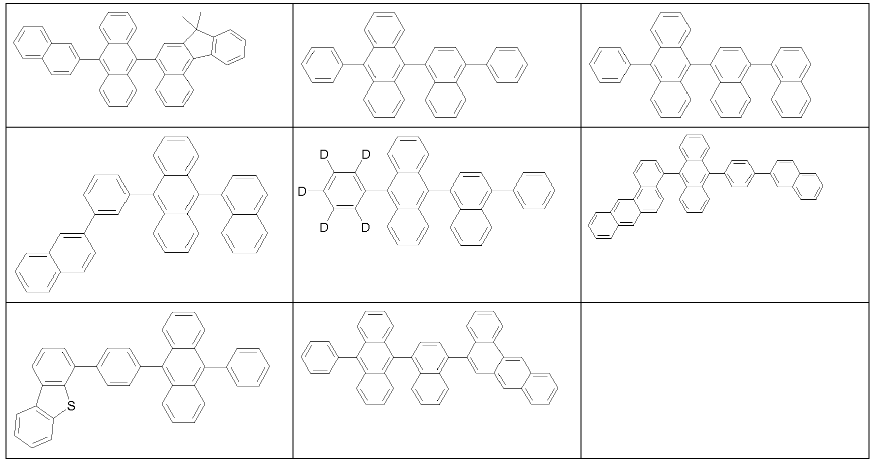

Geeignete Ladungstransportmaterialien, wie sie in der Lochinjektions- bzw. Lochtransportschicht bzw. Elektronenblockierschicht oder in der Elektronentransportschicht der erfindungsgemäßen organischen Elektrolumineszenzvorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Beispiele für bevorzugte Lochtransportmaterialien, die in einer Lochtransport-, Lochinjektions- oder Elektronenblockierschicht in der erfindungsgemäßen Elektrolumineszenzvorrichtung verwendet werden können, sind Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder WO 2013/120577), Fluoren-Amine (z. B. gemäß WO 2014/015937, WO 2014/015938 und WO 2014/015935), Spiro-Dibenzopyran-Amine (z. B. gemäß WO 2013/083216) und Dihydroacridin-Derivate (z. B. gemäß WO 2012/150001).

Als Kathode der organischen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere erfindungsgemäße Verbindungen in Displays, als Lichtquellen in Beleuchtungsanwendungen sowie als Lichtquellen in medizinischen und/oder kosmetischen Anwendungen (z.B. Lichttherapie) eingesetzt werden.

### Ausführungsbeispiele

### A) Synthesebeispiele

### A-1) Synthese der Verbindung 1

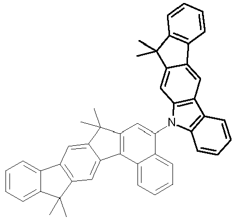

5-Brom-7,13-dihydro-7,7,13,13-tetramethylbenzo[g]indeno[1,2-*b*]fluoren (400 mg, 0.86 mmol, 95.4%ig), 5,7-Dihydro-7,7-dimethyl-indeno-[2,1-b]carbazol (292.4 mg, 1.03 mmol, 1.2 Äquiv.), Pd₂(dba)₃ (16.07 mg, 0.017 mmol, 2 Mol-%) und SPhos (14.12 mg, 0.034 mmol, 4 Mol-%) werden in ein Vial eingewogen, mit Schutzgasatmosphäre versehen, einem Septum verschlossen und mit 8 mL Toluol versetzt. Anschließend wird bei RT K₃PO₄ (583.3 mg, 2.69 mmol, 3.1 Äquiv.) unter Rühren zu der Reaktionsmischung zugegeben. Der Reaktionsansatz wird über Nacht bei 105 °C für 8 d in einem Heizblock unter Rühren erhitzt. Nach Abkühlenlassen auf Raumtemperatur wird die Reaktionslösung mit destilliertem H₂O versetzt und die wässrige Phase mit Toluol extrahiert. Die organische Phase wird über MgSO₄ getrocknet, eingeengt und das Rohprodukt wird durch Säulenchromatographie (Eluent: Heptan : DCM Vol./Vol. 15:1 → 1:1) auf Kieselgel aufgereinigt.

Das Produkt wird als hellgelber Feststoff (78 mg, 14%) erhalten.
MS (EI) m/z berechnet für C₄₉H₃₉N: 641.3, gefunden [M]⁺: 641.4. Elementaranalyse berechnet (%) für C₄₉H₃₉N: C 91.69, H 6.12, N 2.18, gefunden: C 91.62, H 6.55, N 2.07.

### A-2) Synthese der Verbindung 2

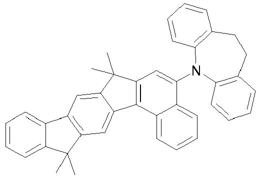

5-Brom-7,13-dihydro-7,7,13,13-tetramethylbenzo[*g*]indeno[1,2-*b*]fluoren (400 mg, 0.86 mmol, 95.4%ig), 11-Dihydro-5H-dibenz[b,f]azepin (184.7 mg, 0.95 mmol, 1.2 Äquiv.), Pd(OAc)₂ (3.93 mg, 0.017 mmol, 2 Mol-%) und SPhos (14.12 mg, 0.034 mmol, 4 Mol-%) werden in ein Vial eingewogen, mit Schutzgasatmosphäre versehen, einem Septum verschlossen und mit 6 mL Toluol versetzt. Anschließend wird bei RT n-Hexyllithium (2.47M in Hexan) (0.39 mL, 0.96 mmol, 1.1 Äquiv.) unter Rühren zu der Reaktionsmischung vorsichtig zugetropft. Der Reaktionsansatz wird über Nacht bei 85 °C für einen Tag in einem Heizblock unter Rühren erhitzt. Nach Abkühlenlassen auf Raumtemperatur wird die Reaktionslösung mit destilliertem H₂O versetzt und die wässrige Phase mit Toluol extrahiert. Die organische Phase wird über MgSO₄ getrocknet, eingeengt und das Rohprodukt wird durch Säulenchromatographie auf Kieselgel (Eluent: Heptan : Toluol Vol./Vol. 2:1 → 1:1 → DCM) aufgereinigt.

Das Produkt wird als hellgelber Feststoff (28 mg, 6%) erhalten.
MS (EI) *m*/*z* berechnet für C₄₂H₃₅N: 553.3, gefunden [*M*]⁺: 553.3. Elementaranalyse berechnet (%) für C₄₂H₃₅N: C 91.10, H 6.37, N 2.53, gefunden: C 89.91, H 7.18, N 2.25.

### A-3) Synthese der Verbindung 3

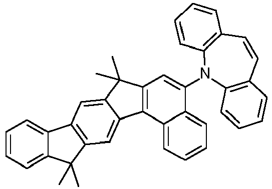

5-Brom-7,13-dihydro-7,7,13,13-tetramethylbenzo[g]indeno[1,2-*b*]fluoren (400 mg, 0.86 mmol, 95.4%ig), 5H-Dibenz[b,f]azepin (199.5 mg, 1.03 mmol, 1.2 Äquiv.), Pd(OAc)₂ (3.93 mg, 0.017 mmol, 2 Mol-%) und SPhos (14.12 mg, 0.034 mmol, 4 Mol-%) werden in ein Vial eingewogen, mit Schutzgasatmosphäre versehen, einem Septum verschlossen und mit 6 mL Toluol versetzt. Anschließend wird bei RT n-Hexyllithium (2.47M in Hexan) (0.39 mL, 0.96 mmol, 1.1 Äquiv.) unter Rühren zu der Reaktionsmischung vorsichtig zugetropft. Der Reaktionsansatz wird über Nacht bei 85 °C für einen Tag in einem Heizblock unter Rühren erhitzt. Nach Abkühlenlassen auf Raumtemperatur wird die Reaktionslösung mit destilliertem H₂O versetzt und die wässrige Phase mit Toluol extrahiert. Die organische Phase wird über MgSO₄ getrocknet, über AlOx (basisch) filtriert und eingeengt. Der erhaltene Rückstand wird mit Acetonitril und 2-Propanol behandelt, der ausgefallene Feststoff filtriert und im Vakuum getrocknet. Es werden 445 mg (93%) des Produktes in Form eines leuchtend gelben Feststoffes erhalten. MS (EI) m/z berechnet für C₄₂H₃₃N: 551.3, gefunden [M]⁺: 551.4. Elementaranalyse berechnet (%) für C₄₂H₃₃N: C 91.43, H 6.03, N 2.54; gefunden: C 91.13, H 6.10, N 2.52.

### B) Device-Beispiele

### Herstellung der OLEDs

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (Schichtdickenvariation, Materialien) angepasst wird.

In den folgenden Beispielen (siehe Tabellen 1 bis 3) werden die Daten verschiedener OLEDs vorgestellt. Als Substrate werden Glassubstrate verwendet, die mit strukturiertem ITO (Indium Zinn Oxid) der Dicke 50 nm beschichtet sind. Die OLEDs haben prinzipiell folgenden Schichtaufbau: Substrat / Buffer / Lochinjektionsschicht (95% HIL1+ 5% HIL2, 20nm)/ Lochtransportschicht (HTL, Dicke in Tabelle 1 angegeben) / Emissionsschicht (EML, 20nm) / Elektronentransportschicht (50% ETL+50% EIL, 20 nm) / Elektroneninjektionsschicht (EIL, 3nm) und abschließend eine Kathode. Die Kathode wird durch eine 100 nm dicke Aluminiumschicht gebildet. Als Buffer wird eine 20nm dicke Schicht Clevios P VP AI 4083 (bezogen von Heraeus Clevios GmbH, Leverkusen) durch Spincoating aufgebracht. Alle restlichen Materialien werden in einer Vakuumkammer thermisch aufgedampft. Der Aufbau der OLEDs ist in Tabelle 1 gezeigt. Die verwendeten Materialien sind in Tabelle 3 gezeigt.

Die Emissionsschicht (EML) besteht immer aus mindestens einem Matrixmaterial (Host=H) und einem emittierenden Dotierstoff (Dotand=D), der dem Matrixmaterial durch Coverdampfung in einem bestimmten Volumenanteil beigemischt wird. Eine Angabe wie H1:D1 (97%:3%) bedeutet hierbei, dass das Material H1 in einem Volumenanteil von 97% und D1 in einem Volumenanteil von 3% in der Schicht vorliegt.

Die OLEDs werden standardmäßig charakterisiert. Hierfür werden die Elektrolumineszenzspektren aufgenommen, die Stromeffizienz (gemessen in cd/A) und die externe Quanteneffizienz (EQE, gemessen in Prozent) in Abhängigkeit der Leuchtdichte unter Annahme einer lambertschen Abstrahlcharakteristik aus Strom-Spannungs-Leuchtdichte-Kennlinien (IUL-Kennlinien) berechnet und abschließend die Lebensdauer der Bauteile bestimmt. Die Elektrolumineszenzspektren werden bei einer Leuchtdichte von 1000 cd/m² aufgenommen und daraus die CIE 1931 x und y Farbkoordinaten berechnet. Die Angabe EQE @ 10 mA/cm² bezeichnet die externe Quanteneffizienz bei einer Betriebsstromdichte von 10 mA/cm². Die Lebensdauer LD95 @ 10 mA/cm² ist die Zeit, die vergeht, bis die Starthelligkeit bei bei einer Betriebsstromdichte von 10 mA/cm² um 5% gesunken ist. Die erhaltenen Daten für die verschiedenen OLEDs sind in Tabelle 2 zusammengefasst.

### Ergebnisse: Verwendung der erfindungsgemäßen Verbindungen als Dotanden in fluoreszierenden OLEDs

Die erfindungsgemäßen Verbindungen eignen sich besonders als blau fluoreszierende Dotanden. Die erfindungsgemäße Verbindung D2 wird in den vorliegenden Beispielen als Emitter in der emittierenden Schicht von OLEDs eingesetzt, jeweils in Kombination mit einem der Hostmaterialien H1 und H2. Als Vergleichsbeispiel wird der Emitter V-D1 vermessen, ebenso jeweils in Kombination mit einem der Hostmaterialien H1 und H2.

Die erhaltenen erfindungsgemäßen OLEDs sind in Tabelle 2 als E3 und E4 bezeichnet. Sie zeigen sehr gute Lebensdauer bei tiefblauer Emission. Verglichen mit dem im Stand der Technik bekannten Emittermaterial V-D1 (vgl. OLEDs V1 und V2 in Tabelle 2) sind sowohl die externe Quanteneffizienz als auch die Lebensdauer stark verbessert, bei tiefblauer Emission.

| | | **Tabelle 1: Aufbau der OLEDs** |
|---|---|---|
| ***Bsp.*** | ***HTL*** | ***EML*** |
| | | *Dicke* / *nm* |
| *V1* | *20nm* | *H1(95%) : V-D1 (5%)*/ *20nm* |
| *V2* | *195nm* | *H2(95%) : V-D1 (5%)* / *20nm* |
| *E3* | *20nm* | *H1 (95%) : D2(5570)* / *20nm* |
| *E4* | *195nm* | *H2(95%) : D2(5570)* / *20nm* |

| **Tabelle 2: Daten der OLEDs** | | | | |
|---|---|---|---|---|
| ***Bsp.*** | ***EQE* @ *10 mA*/*cm²*** *%* | ***LD95* @ *10 mA*/*****cm²**[h]* | ***CIE*** | |
| | | | *x* | *y* |
| *V1* | *6.8* | *55* | 0.149 | 0.123 |
| *V2* | *6.5* | *50* | 0.149 | 0.115 |
| *E3* | *7.5* | *90* | 0.150 | 0.118 |
| *E4* | *7.2* | *80* | 0.149 | 0.121 |

| **Tabelle 3: Strukturen der verwendeten Materialien** | |
|---|---|
| 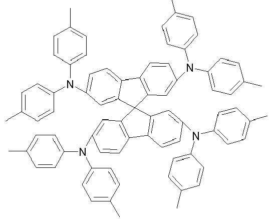 | 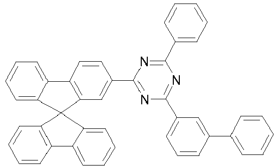 |
| HIL1 | ETL |
| 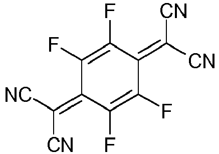 | 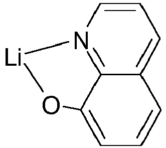 |
| HIL2 | EIL |
| 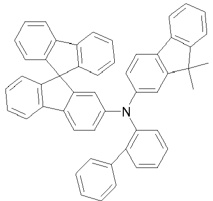 | 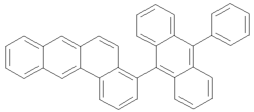 |
| HTL | H1 |
| 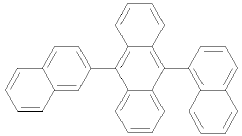 | |
| | 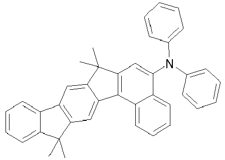 |
| H2 | V-D1 |
| 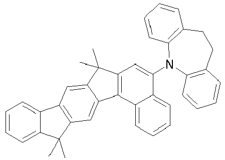 | |
| D2 | |

## Patentansprüche

1. Verbindung gemäß Formel (I) 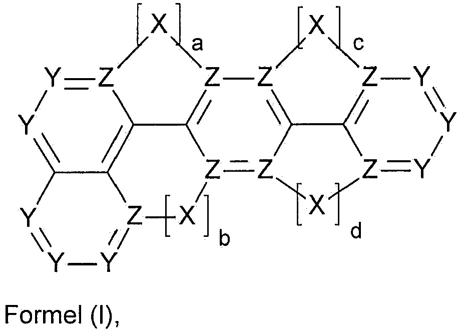 wobei für die auftretenden Variablen gilt:
Y ist bei jedem Auftreten gleich oder verschieden gewählt aus CR¹ und N;
Z ist gleich C, falls an die Gruppe Z eine Brücke X gebunden ist, und ist gleich Y, falls an die Gruppe Z keine Brücke X gebunden ist;
X ist bei jedem Auftreten gleich oder verschieden eine bivalente Brücke, ausgewählt aus C(R¹)₂ und Si(R¹)₂;
R¹ ist bei jedem Auftreten gleich oder verschieden gewählt aus H, D, F, C(=O)R², CN, Si(R²)₃, N(R²)₂, P(=O)(R²)₂, OR², S(=O)R², S(=O)₂R², geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R² substituiert sein können; und wobei eine oder mehrere CH₂-Gruppen in den genannten Alkyl-, Alkoxy-, Alkenyl- und Alkinylgruppen durch -R²C=CR²-, -C=C-, Si(R²)₂, C=O, C=NR², -C(=O)O-, -C(=O)NR²-, NR², P(=O)(R²), -O-, -S-, SO oder SO₂ ersetzt sein können;
R² ist bei jedem Auftreten gleich oder verschieden gewählt aus H, D, F, CN, Alkylgruppen mit 1 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R² miteinander verknüpft sein können und einen Ring bilden können; und wobei die genannten Alkylgruppen, aromatischen Ringsysteme und heteroaromatischen Ringsysteme mit F oder CN substituiert sein können;
a, b, c, d ist bei jedem Auftreten gleich oder verschieden 0 oder 1, mit der Maßgabe, dass a + b = 1 ist und c + d = 1 ist, wobei a = 0 bzw. b = 0 bzw. c = 0 bzw. d = 0 jeweils bedeutet, dass die entsprechende Brücke X nicht vorhanden ist;
wobei mindestens ein Y vorliegt, bei dem anstelle von R¹ eine Einheit der Formel (N) gebunden ist, 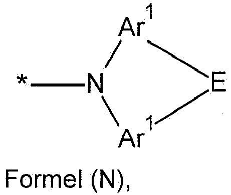 bei der die mit Stern gekennzeichnete Bindung die Bindung an Ymarkiert, und wobei die Formel (N) einer der folgenden Formeln (N-2) bis (N-12) entspricht: 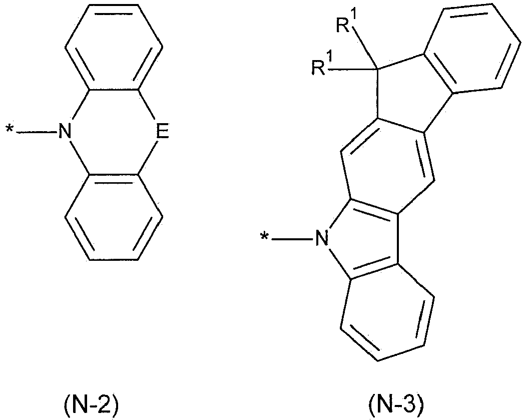 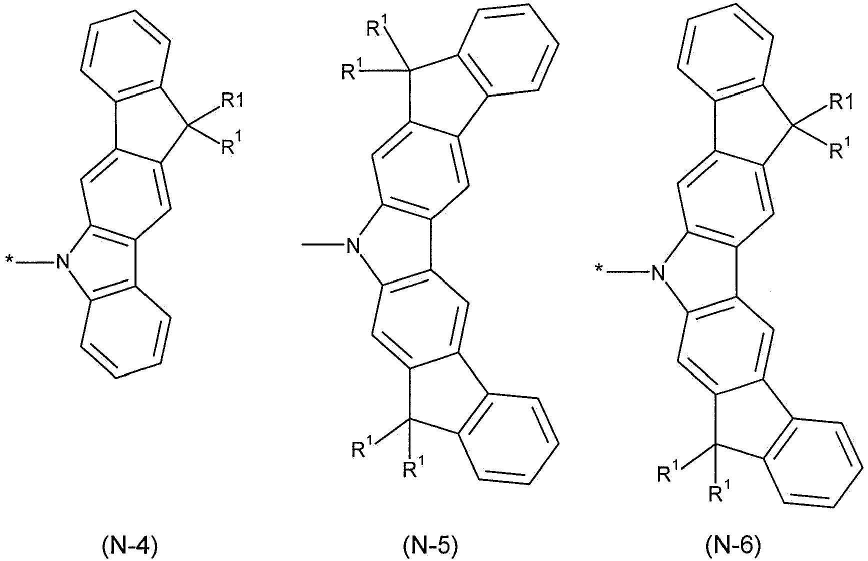 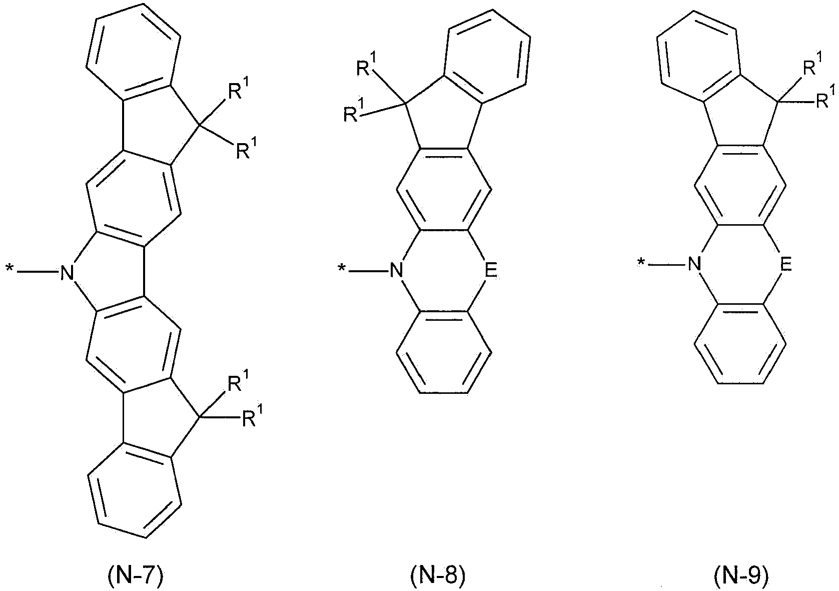 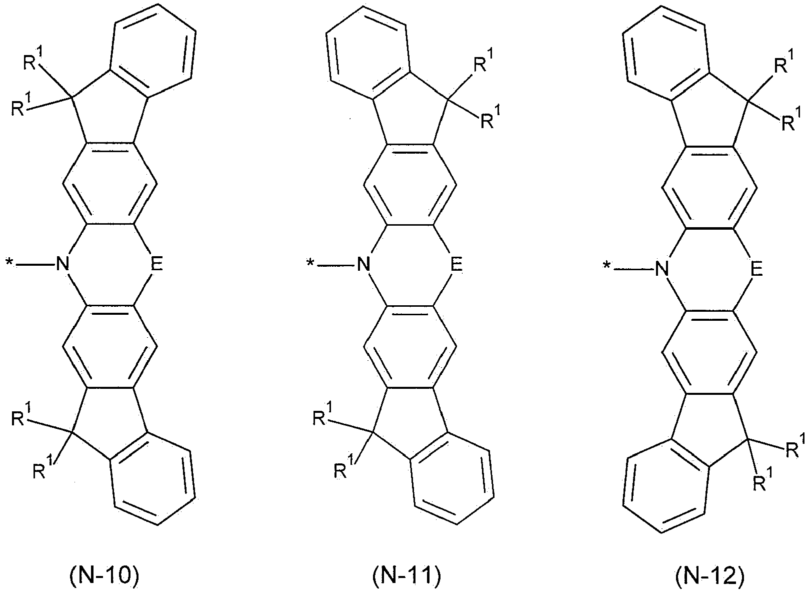 wobei die Einheiten der Formel (N) an allen unsubstituiert gezeichneten Positionen mit Resten R¹ substituiert sein können;
E ist bei jedem Auftreten gleich oder verschieden gewählt aus den folgenden divalenten Gruppen
-C(R¹)₂-C(R¹)₂- (E-2)
-C(R¹)₂-C(R¹)₂-C(R¹)₂- (E-3)
-CR¹=CR¹- (E-4)
wobei R¹ definiert ist wie oben.

2. Verbindung nach Anspruch 1, **dadurch gekennzeichnet, dass** in der Verbindung der Formel (I) genau ein Y vorliegt, bei dem anstelle von R¹ eine Einheit der Formel (N) gebunden ist.

3. Verbindung nach Anspruch 1 oder 2, **dadurch gekennzeichnet, dass** alle Gruppen Y gleich CR¹ sind.

4. Verbindung nach einem oder mehreren der vorangehenden Ansprüche, **dadurch gekennzeichnet, dass** X gleich C(R¹)₂ ist.

5. Verbindung nach einem oder mehreren der vorangehenden Ansprüche, **dadurch gekennzeichnet, dass** Reste R¹ in Einheiten X bei jedem Auftreten gleich oder verschieden gewählt sind aus geradkettigen Alkylgruppen mit 1 bis 12 C-Atomen, verzweigten oder cyclischen Alkylgruppen mit 3 bis 12 C-Atomen, und aromatischen Ringsystemen mit 6 bis 20 aromatischen Ringatomen; wobei die genannten Alkylgruppen und die genannten aromatischen Ringsysteme jeweils mit einem oder mehreren Resten R² substituiert sein können.

6. Verbindung nach einem oder mehreren der vorangehenden Ansprüche, **dadurch gekennzeichnet, dass** Reste R¹ in Einheiten Y bei jedem Auftreten gleich oder verschieden gewählt sind aus H, D, F, CN, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R² substituiert sein können.

7. Verbindung nach einem oder mehreren der vorangehenden Ansprüche, **dadurch gekennzeichnet, dass** Formel (I) einer der folgenden Formeln (I-1-1) bis (I-4-1) entspricht 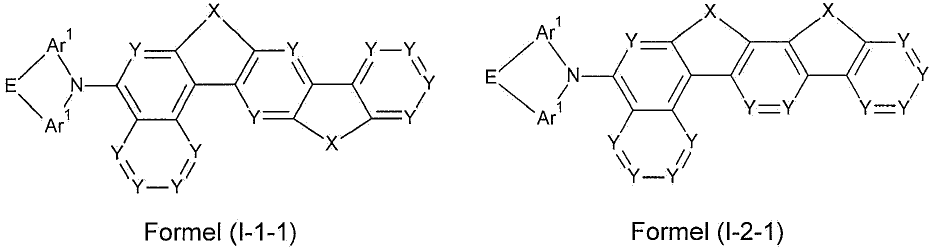 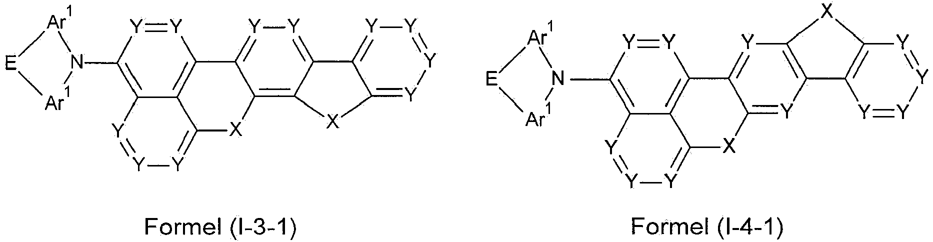 wobei die auftretenden Variablen definiert sind wie in Anspruch 1.

8. Verfahren zur Herstellung einer Verbindung der Formel (I) nach einem oder mehreren der Ansprüche 1 bis 7, **dadurch gekennzeichnet, dass** ein Monobenzindenofluoren-Derivat, welches eine oder mehrere reaktive Gruppen umfasst, in einer übergangsmetallkatalysierten Kupplungsreaktion mit einem Amin umgesetzt wird.

9. Oligomer, Polymer oder Dendrimer, enthaltend eine oder mehrere Verbindungen gemäß Formel (I) nach einem oder mehreren der Ansprüche 1 bis 7, wobei die Bindung(en) zum Polymer, Oligomer oder Dendrimer an beliebigen, in Formel (I) mit R¹ oder R² substituierten Positionen lokalisiert sein können.

10. Formulierung, enthaltend mindestens eine Verbindung nach einem oder mehreren der Ansprüche 1 bis 7 oder ein Polymer nach Anspruch 9, sowie mindestens ein Lösungsmittel.

11. Elektronische Vorrichtung, enthaltend mindestens eine Verbindung nach einem oder mehreren der Ansprüche 1 bis 7, oder ein Polymer nach Anspruch 9.

12. Elektronische Vorrichtung nach Anspruch 11, **dadurch gekennzeichnet, dass** sie eine organische Elektrolumineszenzvorrichtung ist, enthaltend Anode, Kathode und mindestens eine emittierende Schicht, wobei mindestens eine organische Schicht der Vorrichtung, die eine emittierende Schicht, eine Lochtransportschicht oder eine andere Schicht sein kann, die mindestens eine Verbindung oder das Polymer enthält.

13. Elektronische Vorrichtung nach Anspruch 12, **dadurch gekennzeichnet, dass** die mindestens eine organische Schicht gewählt ist aus einer emittierenden Schicht, bevorzugt einer emittierenden Schicht, welche zusätzlich zu der mindestens einen Verbindung oder dem Polymer eine oder mehrere Matrixverbindungen enthält.

14. Verwendung einer Verbindung nach einem oder mehreren der Ansprüche 1 bis 7 in einer elektronischen Vorrichtung.

## Claims

1. Compound of formula (I) 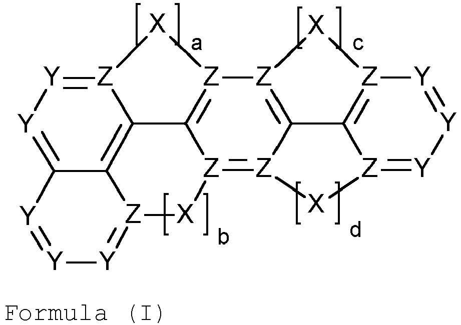 where the variables that occur are as follows:
Y is the same or different at each instance and is selected from CR¹ and N;
Z is C if an X bridge is bonded to the Z group, and is Y if no X bridge is bonded to the Z group;
X is the same or different at each instance and is a bivalent bridge selected from C(R¹)₂ and Si(R¹)₂;
R¹ is the same or different at each instance and is selected from H, D, F, C(=O)R², CN, Si(R²)₃, N(R²)₂, P(=O)(R²)₂, OR², S(=O)R², S(=O)₂R², straight-chain alkyl or alkoxy groups having 1 to 20 carbon atoms, branched or cyclic alkyl or alkoxy groups having 3 to 20 carbon atoms, alkenyl or alkynyl groups having 2 to 20 carbon atoms, aromatic ring systems having 6 to 40 aromatic ring atoms, and heteroaromatic ring systems having 5 to 40 aromatic ring atoms; where two or more R¹ radicals may be joined to one another and may form a ring; where the alkyl, alkoxy, alkenyl and alkynyl groups mentioned and the aromatic ring systems and heteroaromatic ring systems mentioned may each be substituted by one or more R² radicals; and where one or more CH₂ groups in the alkyl, alkoxy, alkenyl and alkynyl groups mentioned may be replaced by -R²C=CR²-, -C≡C-, Si(R²)₂, C=O, C=NR², -C(=O)O-, C(=O)NR²-, NR², P(=O)(R²), -O-, -S-, SO or SO₂;
R² is the same or different at each instance and is selected from H, D, F, CN, alkyl groups having 1 to 20 carbon atoms, aromatic ring systems having 6 to 40 aromatic ring atoms and heteroaromatic ring systems having 5 to 40 aromatic ring atoms; where two or more R² radicals may be joined to one another and may form a ring; and where the alkyl groups, aromatic ring systems and heteroaromatic ring systems mentioned may be substituted by F or CN;
a, b, c, d is the same or different at each instance and is 0 or 1, with the proviso that a + b = 1 and c + d = 1, where a = 0, b = 0, c = 0 and d = 0 respectively means that the corresponding X bridge is absent;
where there is at least one Y in which a unit of the formula (N) is bonded instead of R¹, 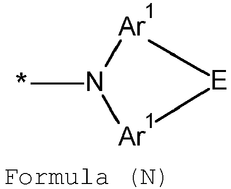 in which the bond identified by an asterisk marks the bond to Y, and where the formula (N) corresponds to one of the following formulae (N-2) to (N-12): 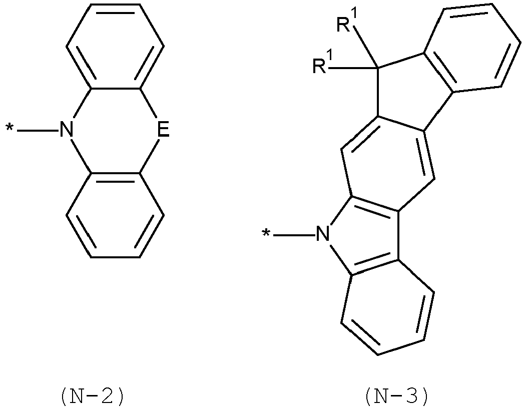 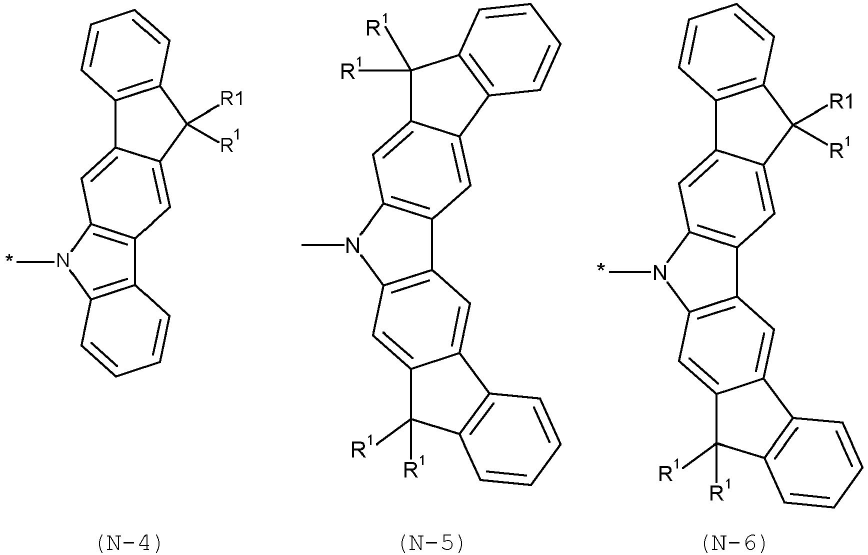 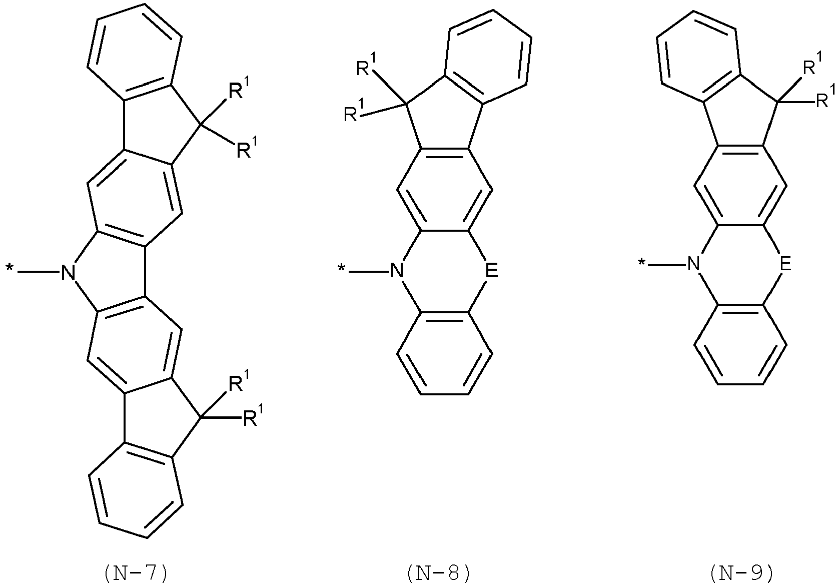 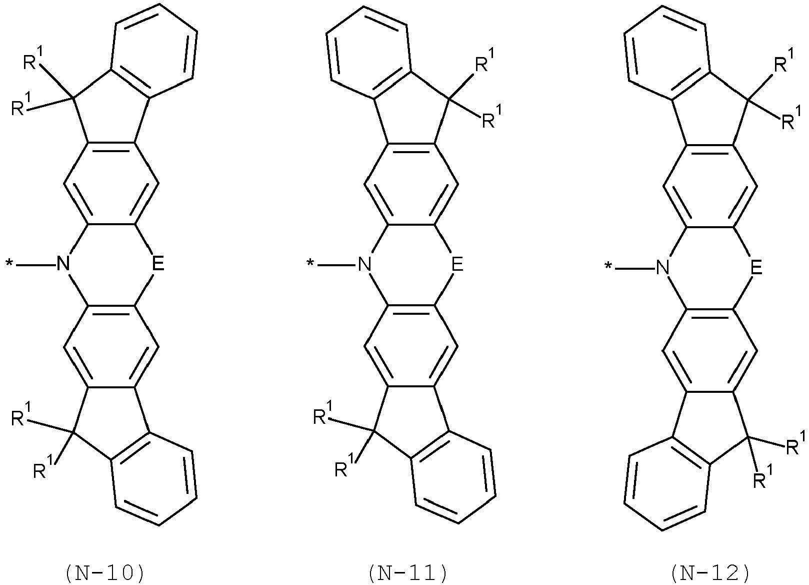 where the units of the formula (N) may be substituted at all positions shown as unsubstituted by R¹ radicals;
E is the same or different at each instance and is selected from the following divalent groups:
-C(R¹)₂-C(R¹)₂- (E-2)
-C(R¹)₂-C(R¹)₂- (E-3)
₂-C(R¹)-CR¹=CR¹- (E-4)
where R¹ is as defined above.

2. Compound according to Claim 1, **characterized in that**, in the compound of the formula (I), there is exactly one Y in which a unit of the formula (N) is bonded instead of R¹.

3. Compound according to Claim 1 or 2, **characterized in that** all Y groups are CR¹.

4. Compound according to one or more of the preceding claims, **characterized in that** X is C(R¹)₂.

5. Compound according to one or more of the preceding claims, **characterized in that** R¹ radicals in X units are the same or different at each instance and are selected from straight-chain alkyl groups having 1 to 12 carbon atoms, branched or cyclic alkyl groups having 3 to 12 carbon atoms, and aromatic ring systems having 6 to 20 aromatic ring atoms; where the alkyl groups mentioned and the aromatic ring systems mentioned may each be substituted by one or more R² radicals.

6. Compound according to one or more of the preceding claims, **characterized in that** R¹ radicals in Y units are the same or different at each instance and are selected from H, D, F, CN, straight-chain alkyl groups having 1 to 20 carbon atoms, branched or cyclic alkyl groups having 3 to 20 carbon atoms, aromatic ring systems having 6 to 40 aromatic ring atoms, and heteroaromatic ring systems having 5 to 40 aromatic ring atoms; where the alkyl groups mentioned, the aromatic ring systems mentioned and the heteroaromatic ring systems mentioned may each be substituted by one or more R² radicals.

7. Compound according to one or more of the preceding claims, **characterized in that** formula (I) corresponds to one of the following formulae (I-1-1) to (1-4-1): 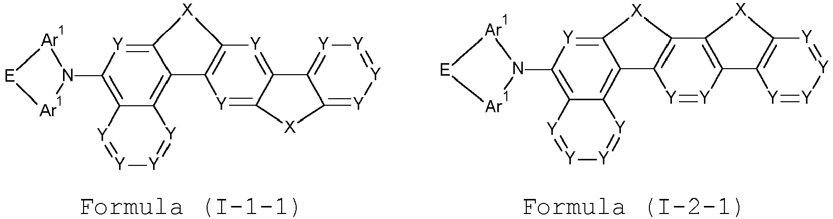 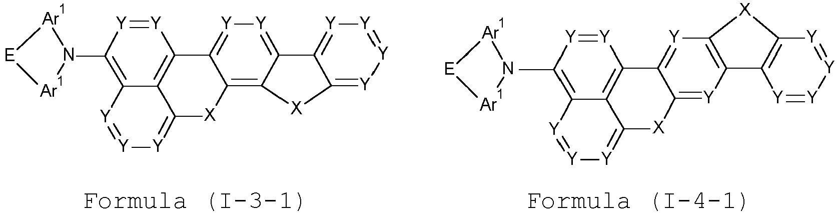 where the variables that occur are as defined in Claim 1.

8. Process for preparing a compound of the formula (I) according to one or more of Claims 1 to 7, **characterized in that** a monobenzoindenofluorene derivative comprising one or more reactive groups is reacted with an amine in a transition metal-catalysed coupling reaction.

9. Oligomer, polymer or dendrimer containing one or more compounds of formula (I) according to one or more of Claims 1 to 7, wherein the bond(s) to the polymer, oligomer or dendrimer may be localized at any desired positions substituted by R¹ or R² in formula (I).

10. Formulation comprising at least one compound according to one or more of Claims 1 to 7 or a polymer according to Claim 9, and at least one solvent.

11. Electronic device comprising at least one compound according to one or more of Claims 1 to 7 or a polymer according to Claim 9.

12. Electronic device according to Claim 11, **characterized in that** it is an organic electroluminescent device comprising anode, cathode and at least one emitting layer, where at least one organic layer of the device, which may be an emitting layer, a hole transport layer or another layer, contains the at least one compound or the polymer.

13. Electronic device according to Claim 12, **characterized in that** the at least one organic layer is selected from an emitting layer, preferably an emitting layer which, in addition to the at least one compound or the polymer, comprises one or more matrix compounds.

14. Use of a compound according to one or more of Claims 1 to 7 in an electronic device.

## Revendications

1. Composé selon la formule (I) 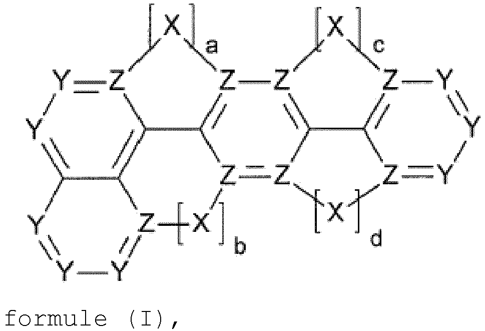 ce qui suit s'appliquant aux variables apparaissant :
Y, identique ou différent en chaque occurrence, étant choisi parmi CR¹ et N ;
Z étant égal à C, dans le cas où un pont X est lié au groupe Z, et étant égal à Y, dans le cas où aucun pont X n'est lié au groupe Z ;
X, identique ou différent en chaque occurrence, étant un pont divalent choisi parmi C(R¹)₂ et Si(R¹)₂ ;
R¹, identique ou différent en chaque occurrence, étant choisi parmi H, D, F, C(=O)R², CN, Si(R²)₃, N(R²)₂, P(=O) (R²)₂, OR², S(=O)R², S(=O)₂R², des groupes alkyle ou alcoxy linéaires comportant 1 à 20 atomes de C, des groupes alkyle ou alcoxy ramifiés ou cycliques comportant 3 à 20 atomes de C, des groupes alcényle ou alcynyle comportant 2 à 20 atomes de C, des systèmes cycliques aromatiques comportant 6 à 40 atomes de cycle aromatiques, et des systèmes cycliques hétéroaromatiques comportant 5 à 40 atomes de cycle aromatiques ; deux radicaux R¹ ou plus pouvant être reliés l'un avec l'autre et pouvant former un cycle ; les groupes alkyle, alcoxy, alcényle et alcynyle mentionnés et les systèmes cycliques aromatiques et les systèmes cycliques hétéroaromatiques mentionnés pouvant à chaque fois être substitués par un ou plusieurs radicaux R² ; et un ou plusieurs groupes CH₂ dans les groupes alkyle, alcoxy, alcényle et alcynyle mentionnés pouvant être remplacés par -R²C=CR²-, -C≡C-, Si(R²)₂, C=O, C=NR², -C(=O)O-, -C(=O)NR²-, NR², P(=O) (R²), -O-, -S-, SO ou SO₂ ;
R², identique ou différent en chaque occurrence, étant choisi parmi H, D, F, CN, des groupes alkyle comportant 1 à 20 atomes de C, des systèmes cycliques aromatiques comportant 6 à 40 atomes de cycle aromatiques et des systèmes cycliques hétéroaromatiques comportant 5 à 40 atomes de cycle aromatiques ; deux radicaux R² ou plus pouvant être reliés l'un avec l'autre et pouvant former un cycle ; et les groupes alkyle, les systèmes cycliques aromatiques et les systèmes cycliques hétéroaromatiques mentionnés pouvant être substitués par F ou CN ;
a, b, c, d, identiques ou différents en chaque occurrence, étant 0 ou 1, étant entendu que a + b = 1 et c + d = 1, avec a = 0 ou b = 0 ou c = 0 ou d = 0 signifiant à chaque fois que le pont correspondant X n'est pas présent ;
au moins un Y étant présent dans lequel au lieu de R¹, un motif de formule (N) est lié, 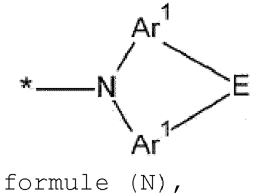 dans laquelle la liaison indiquée par l'étoile marque la liaison à Y, et la formule (N) correspondant à une des formules suivantes (N-2) à (N-12) : 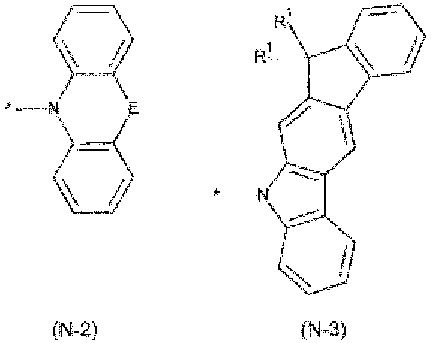 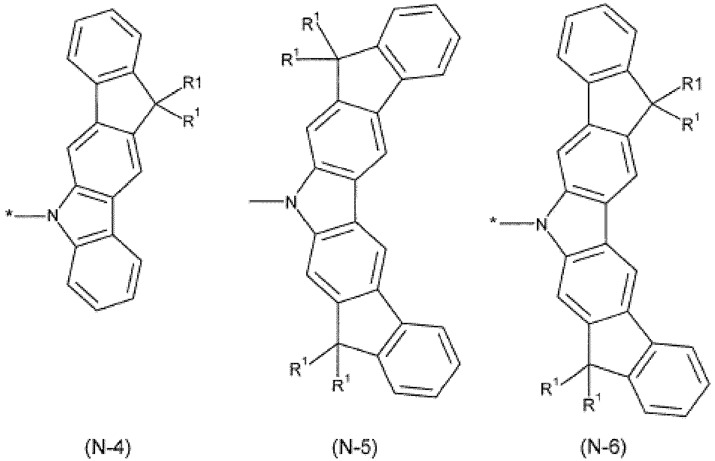 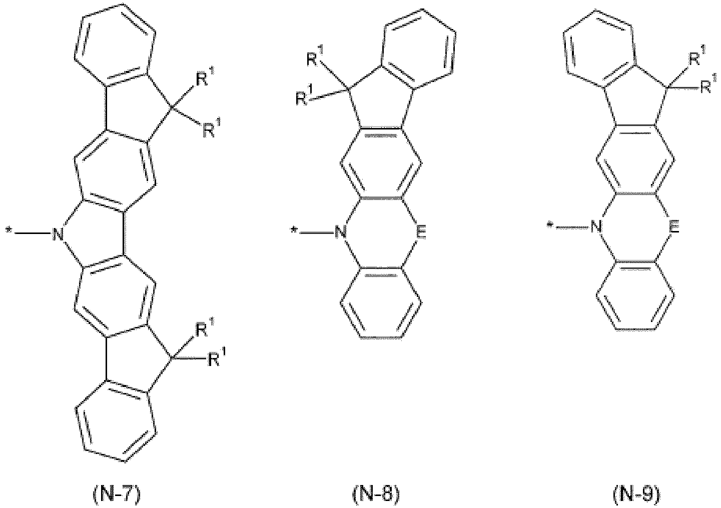 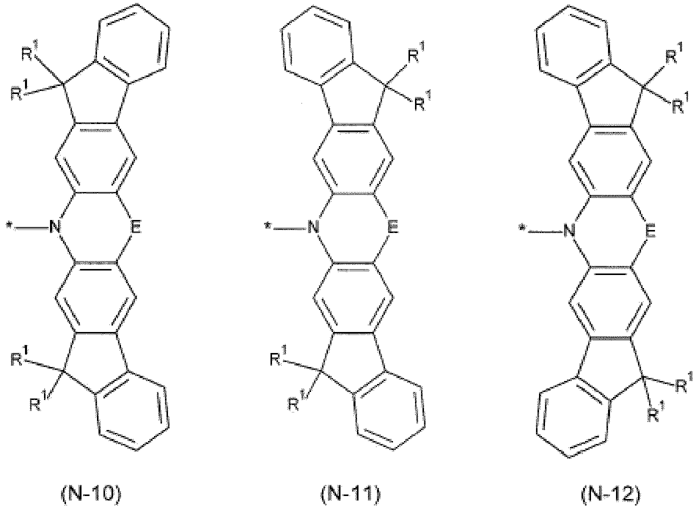 les motifs de formule (N) pouvant être substitués par des radicaux R¹ au niveau de toutes les positions représentées de manière non substituée ;
E, identique ou différent en chaque occurrence, étant choisi parmi les groupes divalents suivants
-C(R¹)₂-C(R¹)₂- (E-2)
-C(R¹)₂-C(R¹)₂-C(R¹)₂- (E-3)
-CR¹=CR¹- (E-4),
R¹ étant défini comme ci-dessus.

2. Composé selon la revendication 1, **caractérisé en ce que** dans le composé de formule (I) exactement un Y est présent dans lequel, au lieu de R¹, un motif de formule (N) est lié.

3. Composé selon la revendication 1 ou 2, **caractérisé en ce que** tous les groupes Y sont égaux à CR¹.

4. Composé selon l'une ou plusieurs des revendications précédentes, **caractérisé en ce que** X est égal à C(R¹)₂.

5. Composé selon l'une ou plusieurs des revendications précédentes, **caractérisé en ce que** des radicaux R¹, identiques ou différents en chaque occurrence, dans des motifs X sont choisis parmi des groupes alkyle linéaires comportant 1 à 12 atomes de C, des groupes alkyle ramifiés ou cycliques comportant 3 à 12 atomes de C et des systèmes cycliques aromatiques comportant 6 à 20 atomes de cycle aromatiques ; les groupes alkyle mentionnés et les systèmes cycliques aromatiques mentionnés pouvant à chaque fois être substitués par un ou plusieurs radicaux R².

6. Composé selon l'une ou plusieurs des revendications précédentes, **caractérisé en ce que** des radicaux R¹, identiques ou différents en chaque occurrence, dans des motifs Y sont choisis parmi H, D, F, CN, des groupes alkyle linéaires comportant 1 à 20 atomes de C, des groupes alkyle ramifiés ou cycliques comportant 3 à 20 atomes de C, des systèmes cycliques aromatiques comportant 6 à 40 atomes de cycle aromatiques et des systèmes cycliques hétéroaromatiques comportant 5 à 40 atomes de cycle aromatiques ; les groupes alkyle mentionnés, les systèmes cycliques aromatiques mentionnés et les systèmes cycliques hétéroaromatiques mentionnés pouvant à chaque fois être substitués par un ou plusieurs radicaux R².

7. Composé selon l'une ou plusieurs des revendications précédentes, **caractérisé en ce que** la formule (I) correspond à une des formules suivantes (I-1-1) à (I-4-1) 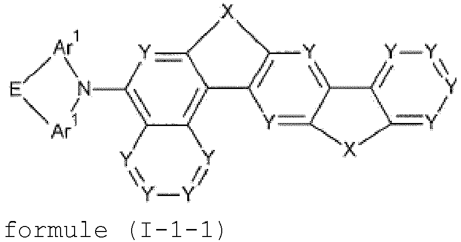 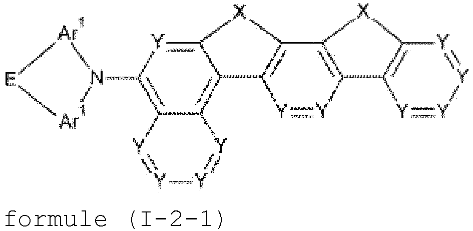 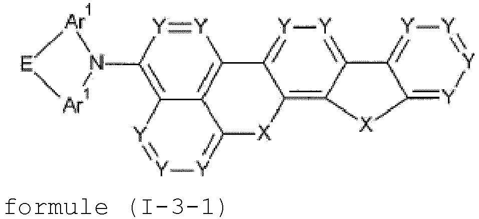 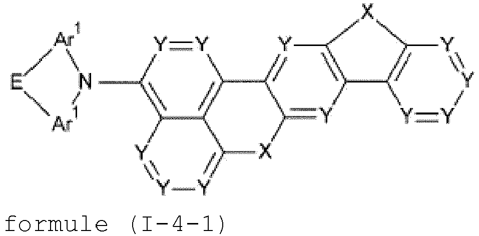 les variables apparaissant étant définies comme dans la revendication 1.

8. Procédé pour la préparation d'un composé de formule (I) selon l'une ou plusieurs des revendications 1 à 7, **caractérisé en ce qu'**un dérivé de type monobenzindénofluorène, qui comprend un ou plusieurs groupes réactifs, est transformé avec une amine dans une réaction de couplage catalysée par un métal de transition.

9. Oligomère, polymère ou dendrimère, contenant un ou plusieurs composés selon la formule (I) selon l'une ou plusieurs des revendications 1 à 7, la ou les liaison(s) au polymère, à l'oligomère ou au dendrimère pouvant être localisée (s) au niveau de quelconques positions substituées par R¹ ou R² dans la formule (I).

10. Formulation, contenant au moins un composé selon l'une ou plusieurs des revendications 1 à 7 ou un polymère selon la revendication 9, ainsi qu'au moins un solvant.

11. Dispositif électronique, contenant au moins un composé selon l'une ou plusieurs des revendications 1 à 7 ou un polymère selon la revendication 9.

12. Dispositif électronique selon la revendication 11, **caractérisé en ce qu'**il s'agit d'un dispositif organique d'électroluminescence, contenant anode, cathode et au moins une couche émettrice, au moins une couche organique du dispositif, qui peut être une couche émettrice, une couche de transport de trous ou une autre couche, contenant l'au moins un composé ou le polymère.

13. Dispositif électronique selon la revendication 12, **caractérisé en ce que** l'au moins une couche organique est choisie parmi une couche émettrice, préférablement une couche émettrice qui contient, en plus de l'au moins un composé ou du polymère, un ou plusieurs composés de matrice.

14. Utilisation d'un composé selon l'une ou plusieurs des revendications 1 à 7 dans un dispositif électronique.
